# EUROPEAN PATENT APPLICATION

(11) **EP 4 501 211 A1**
(43) Date of publication of application: **05.02.2025**
(21) Application number: 22935741.3
(22) Date of filing: 21.12.2022
(51) Int. Cl.: A61B 3/10

(54) **FUNDUS OBSERVATION DEVICE**

(30) Priority: 31.03.2022 JP 2022057912
(71) Applicant: TOPCON CORPORATION, Tokyo 174-8580 (JP)
(72) Inventor: NAKANISHI, Michiko, Tokyo 174-8580 (JP); UENO, Shingo, Tokyo 174-8580 (JP); TAJIMA, Hiroto, Tokyo 174-8580 (JP)
(74) Representative: Gagel, Roland
(86) International application number: PCT/JP2022/047116
(87) International publication number: WO 2023/188614

(57) **Abstract**

This fundus observation device includes an optical system, two concave mirrors, and a holding member. The optical system projects light from a light source onto the fundus of the subject's eye and receives light returned from the fundus. The two concave mirrors each have a concave reflecting surface, guide light from the optical system to the fundus, and guide return light to the optical system. The holding member can hold the two concave mirrors arranged on both sides so as to have predetermined relative positions in a predetermined one-dimensional direction or predetermined two-dimensional directions.

## Description

### [TECHNICAL FIELD]

The present invention relates to a fundus observation apparatus.

### [BACKGROUND ART]

There is a demand for fundus observation apparatuses capable of easily observing and imaging a fundus or the like of an eye to be examined with a wide field of view for screening or treating eye diseases. Specifically, there is a demand for fundus observation apparatuses that can observe the fundus of the eye to be examined at a wide angle of view of more than 80 degrees in a single imaging (shot). As such fundus observation apparatuses, scanning laser ophthalmoscopes (SLOs) are known. SLO is an apparatus configured to form an image of the fundus by scanning the fundus with light to detect returning light from the fundus with a light receiving device.

Various techniques relate to such a fundus observation apparatus have been proposed.

For example, Patent Document 1 discloses a scanning ophthalmoscope that can scan a retina at a wide angle by moving a two-dimensional collimated light scan using two ellipsoidal mirrors and a plane mirror by using a scan movement means.

### [PRIOR ART DOCUMENTS]

### [PATENT DOCUMENTS]

[PATENT DOCUMENT 1] Japanese Unexamined Patent Application Publication (Translation of PCT Application) No. 2009-543585

### [SUMMARY OF THE INVENTION]

### [PROBLEMS TO BE SOLVED BY THE INVENTION]

In case of observing (photographing) the fundus at a wide angle, light deflected over a wide deflection angle range must be allowed to enter the eye through the pupil. This means that the accuracy of the optical system, such as the ellipsoidal mirror, has a greater impact on the accuracy of fundus observation.

However, in the conventional techniques, even if the optical members themselves, such as ellipsoidal mirrors, are high-precision, the precision of fundus observation is reduced due to the precision of adjustment such as positioning of the optical members, and the adjustment work such as positioning of the optical members is complicated. Thus, a new technique for easily adjusting an optical member with high precision is required.

The present invention has been made in view of such circumstances, and one of objects of the present invention is to provide a new technique for easily adjusting an optical member with high precision.

### [MEANS OF SOLVING THE PROBLEMS]

One aspect of embodiments is a fundus observation apparatus. The fundus observation apparatus includes: an optical system configured to project light from a light source onto a fundus of an eye to be examined, and to receive returning light from the fundus; two concave mirrors having concave reflective surfaces, respectively, configured to guide the light from the optical system to the fundus, and to guide the returning light to the optical system; and a holding member configured to be capable of holding the two concave mirrors in a state where the concave mirrors are arranged on both sides of the holding member so that the concave mirrors are positioned in a default relative position in a predetermined one-dimensional direction or predetermined two-dimensional directions.

### [EFFECTS OF THE INVENTION]

According to the present invention, a new technique for easily adjusting an optical member with high precision can be provided.

### [BRIEF EXPLANATION OF THE DRAWINGS]

[FIG. 1] FIG. 1 is a schematic diagram illustrating an example of a configuration of an optical system of a fundus observation apparatus according to a first embodiment.
[FIG. 2A] FIG. 2A is a schematic diagram illustrating an example of a configuration of a first ellipsoidal mirror and a second ellipsoidal mirror according to the first embodiment.
[FIG. 2B] FIG. 2B is a schematic diagram illustrating an example of a configuration of the first ellipsoidal mirror and the second ellipsoidal mirror according to the first embodiment.
[FIG. 2C] FIG. 2C is a schematic diagram illustrating an example of a configuration of the first ellipsoidal mirror and the second ellipsoidal mirror according to the first embodiment.
[FIG. 2D] FIG. 2D is a schematic diagram illustrating an example of a configuration of the first ellipsoidal mirror and the second ellipsoidal mirror according to the first embodiment.
[FIG. 3] FIG. 3 is a schematic diagram illustrating an example of a configuration of the first ellipsoidal mirror and the second ellipsoidal mirror according to the first embodiment.
[FIG. 4A] FIG. 4A is a schematic diagram illustrating an example of a configuration of the first ellipsoidal mirror and the second ellipsoidal mirror according to a comparative example of the first embodiment.
[FIG. 4B] FIG. 4B is a schematic diagram illustrating an example of a configuration of the first ellipsoidal mirror and the second ellipsoidal mirror according to the comparative example of the first embodiment.
[FIG. 5A] FIG. 5A is a schematic diagram illustrating an example of a configuration of the first ellipsoidal mirror and the second ellipsoidal mirror according to the first embodiment.
[FIG. 5B] FIG. 5B is a schematic diagram illustrating an example of a configuration of the first ellipsoidal mirror and the second ellipsoidal mirror according to the first embodiment.
[FIG. 6] FIG. 6 is a schematic diagram illustrating an example of a configuration of a processing system of the fundus observation apparatus according to the first embodiment.
[FIG. 7] FIG. 7 is a flowchart illustrating an example of an operation of the fundus observation apparatus according to the first embodiment.
[FIG. 8] FIG. 8 is a schematic diagram illustrating an example of a configuration of an optical system of a fundus observation apparatus according to a second embodiment.
[FIG. 9] FIG. 9 is a schematic diagram illustrating an example of a configuration of an optical system of a fundus observation apparatus according to the second embodiment.
[FIG. 10] FIG. 10 is a schematic diagram illustrating an example of a configuration of a processing system of the fundus observation apparatus according to the second embodiment.
[FIG. 11] FIG. 11 is a flowchart illustrating an example of an operation of the fundus observation apparatus according to the second embodiment.
[FIG. 12] FIG. 12 is a schematic diagram for explaining an operation of the fundus observation apparatus according to the second embodiment.
[FIG. 13] FIG. 13 is a schematic diagram illustrating an example of a configuration of an optical system of a fundus observation apparatus according to a third embodiment.
[FIG. 14] FIG. 14 is a schematic diagram illustrating an example of a configuration of an optical system of a fundus observation apparatus according to a fourth embodiment.
[FIG. 15] FIG. 15 is a schematic diagram illustrating an example of a configuration of an optical system of a fundus observation apparatus according to a fifth embodiment.

### [MODES FOR CARRYING OUT THE INVENTION]

Referring now to the drawings, exemplary embodiments of a fundus observation apparatus according to the present invention are described below. Any of the contents of the documents cited in the present specification and arbitrary known techniques may be applied to the embodiments below.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

The fundus observation apparatus according to embodiments includes an optical system and two concave mirrors having concave reflective surfaces, respectively. The fundus observation apparatus is configured to guide light from the optical system to the fundus and to guide returning light from the fundus to the optical system. The optical system is configured to project the light from the light source onto the fundus through the two concave mirrors, and to receive the returning light from the fundus through the two concave mirrors. The fundus observation apparatus includes a holding member. The holding member is configured to be capable of holding the two concave mirrors in a state where the concave mirrors are arranged on both sides of the holding member so that the concave mirrors are positioned in a default relative position in a predetermined one-dimensional direction or predetermined two-dimensional directions. In some embodiments, the holding member is configured to hold the two concave mirrors on both sides of the holding member at a default distance in a predetermined direction. Examples of a predetermined direction include an optical axis direction of the optical system, a scan center direction of the light from the light source, and a traveling direction of the light from the light source.

This allows the two concave mirrors held by the holding member to be fixed in a predetermined positional relationship for one-dimensional direction or two-dimensional directions among three-dimensional directions, thereby reducing the number of directions for which the positional relationship of the two concave mirrors should be adjusted. For example, when the two concave mirrors are fixed in a default positional relationship in the optical axis direction of the optical system, the two concave mirrors need merely to be adjusted in the two-dimensional directions intersecting the optical axis direction of the optical system. As a result, the accuracy of fundus observation can be improved at a low cost while easily achieving optical measurement conditions designed for high precision. In particular, by manufacturing optical members such as concave mirrors using high-precision machining techniques, the deterioration of optical measurement accuracy caused by positioning accuracy can be suppressed.

The optical system includes an imaging optical system, an Optical Coherence Tomography (hereinafter referred to as OCT) optical system, an SLO optical system, or an optical system obtained by combining two or more of these. The imaging optical system is configured to illuminate the fundus with the light from the light source and to form a fundus image based on light receiving result(s) acquired by receiving the returning light from the fundus. The OCT optical system is configured to split light from an OCT light source into measurement light and reference light, to scan the fundus with the measurement light, and to form a fundus image (tomographic image, front image) based on a detection result of interference light acquired by detecting the interference light between returning light of the measurement light from the fundus and the reference light. The SLO optical system is configured to scan the fundus with light from a light source and to form a fundus image based on a light receiving result acquired by receiving returning light from the fundus.

In some embodiments, the two concave mirrors are arranged so that reflective surfaces of the two concave mirrors face each other, and the two concave mirrors are configured to reflect the light deflected by the deflecting member on one of the two concave mirrors and to reflect the reflected light on the other of the two concave mirrors to guide the reflected light to the eye to be examined. In some embodiments, an optical scanner or a reflective mirror is arranged between the two concave mirrors and the optical system is configured to deflect the light reflected on the one of the two concave mirrors using the optical scanner or the reflective mirror, and to reflect the reflected light on the other of the two concave mirrors to guide the reflected light to the eye to be examined.

The concave mirror may be an ellipsoidal mirror whose reflective surface forms a part of an elliptical surface (ellipsoid), a parabolic mirror whose reflective surface forms a part of a paraboloidal surface, or a free-form surface mirror whose reflective mirror forms a part of a free-form surface. The two concave mirrors may be a combination of concave mirrors of same type described above or a combination of concave mirrors of different types from each other described above. In some embodiments, the two concave mirrors are two ellipsoidal mirrors. In some embodiments, the two concave mirrors are the ellipsoidal mirror and the free-form surface mirror.

The fundus observation apparatus, which is configured so that the holding member holds the two concave mirrors as described above, is capable of illuminating the fundus of the eye to be examined with illumination light whose cross-sectional luminous flux shape is a slit-shaped (line-shaped), and of receiving returning light of the illumination light from the fundus using a two-dimensional image sensor on a light receivable range (focal plane, virtual opening range) movable at a position substantially conjugate optically to the fundus, for example. In this case, the fundus can be scanned with the illumination light by coupling (separating) an optical path of the illumination light and an optical path of the returning light and deflecting the illumination light in synchronization with a movement of the light receivable range (focal plane), using a deflecting member. Here, the deflecting member has a configuration that allows the returning light to be transmitted through (pass through) a central part of the deflecting member and to reflect the illumination light on a peripheral part of the central part. In some embodiments, an optical path coupling part between the optical path of the illumination light and the optical path of the returning light in the deflecting member is positioned at a position substantially conjugate optically to a pupil of the eye to be examined. In some embodiments, the deflecting member is a hole mirror.

This allows to avoid the deterioration of the optical measurement conditions caused by the assembly (mounting) accuracy of the optical members with simplicity and low cost and to secure an imaging (shooting) angle of view of more than 80 degrees alone with an optical system configured to scan in a width direction of the line of the illumination light. As a result, a shared optical system between the optical path of the wide-angle illumination light and the optical path of the returning light can be easily arranged.

In some embodiments, the fundus observation apparatus further includes an OCT optical system including an optical scanner. Here, the OCT optical system is configured to irradiate measurement light deflected by the optical scanner onto the eye to be examined and to perform OCT scan that detects interference light between returning light of the measurement light and reference light. In this case, the optical path of the returning light of the slit-shaped (line-shaped) illumination light and an optical path of the OCT optical system are coupled by an optical path coupling separating member arranged between the deflecting member and the two-dimensional image sensor. In other words, by optically coupling the OCT optical system with the optical path of the returning light on the transmission side of the deflecting member (through a hole in the hole mirror), the optical path of the returning light of the illumination light can be separated from the above shared optical system at low cost. Further, OCT measurement (OCT imaging) can be performed on any position on the fundus being observed at a large wide angle, without sharing an optical scanner for OCT scanning and an optical scanner for deflection of illumination light.

Hereinafter, a case where the fundus observation apparatus according to the embodiments acquires images of the fundus of the eye to be examined using two ellipsoidal mirrors (concave mirrors in a broad sense), which are held on both sides of the holding member at a default distance in the optical axis direction of the optical system so as to share one focal point, will be mainly described.

Hereinafter, for convenience of explanation, a scan center direction of the light projected onto the fundus is assumed to be a z direction (optical axis direction of the optical system), an up-down direction (vertical direction) orthogonal to the z direction is assumed to be a y direction, and a left-right direction (horizontal direction) orthogonal to the z direction is assumed to be an x direction.

### <First embodiment>

### <Configuration>

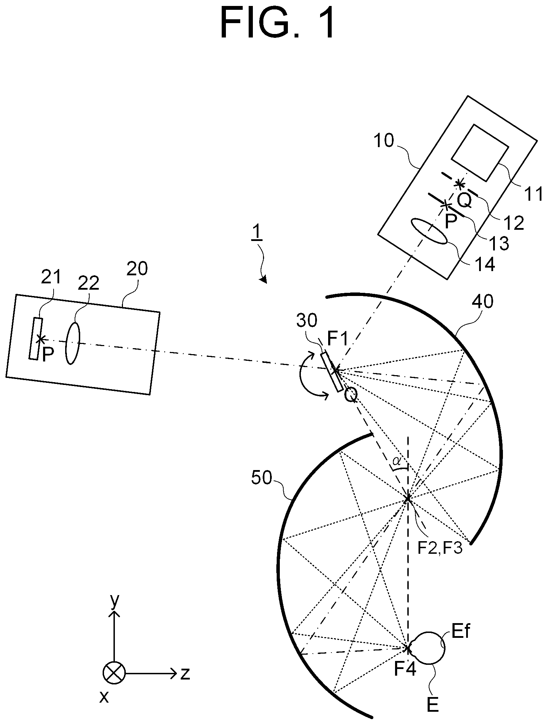

FIG. 1 illustrates an example of a configuration of an optical system of the fundus observation apparatus according to a first embodiment. It should be noted that, in FIG. 1, a position substantially conjugate optically to a fundus Ef of an eye E to be examined is illustrated as a fundus conjugate position P, and a position substantially conjugate optically to a pupil (iris) of the eye E to be examined is illustrated as a pupil conjugate position (iris conjugate position) Q.

The fundus observation apparatus 1 according to the first embodiment includes a slit projection optical system 10, a slit light receiving optical system 20, a hole mirror 30 as a deflecting member having a scanning mechanism, a first ellipsoidal mirror 40, and a second ellipsoidal mirror 50.

### (Slit projection optical system 10)

The slit projection optical system 10 is configured to generate slit-shaped illumination light (illumination light whose luminous flux cross-sectional shape is a line-shaped), and to project the generated illumination light onto the hole mirror 30. The slit projection optical system 10 includes an illumination light source 11, an iris aperture 12, a slit 13, and a projection lens 14.

The illumination light source 11 includes a visible light source that generates light in the visible region. For example, the illumination light source 11 generates light having a central wavelength in the wavelength range of 420 nm to 700 nm. This type of illumination light source 11 includes, for example, an LED (Light Emitting Diode), an LD (Laser Diode), a halogen lamp, or a xenon lamp. In some embodiments, the illumination light source 11 includes a white light source or a light source capable of outputting light with each color component of RGB. In some embodiments, the illumination light source 11 includes a light source capable of switching to output the light in infrared region or the light in visible region. The illumination light source 11 is arranged at a position non-conjugate optically to the fundus Ef of the eye E to be examined and an iris of the eye E to be examined, respectively.

The iris aperture 12 (specifically, aperture(s) described below) can be arranged at the pupil conjugate position Q. In the iris aperture 12, one or more apertures are formed at position(s) away from an optical axis of an optical path of light output from the illumination light source 11. The aperture formed in the iris aperture 12 defines an incident position (incident shape) of the illumination light on the iris of the eye E to be examined. For example, apertures are formed at point-symmetric positions centered on the optical axis. Thereby, when the pupil center of the eye E to be examined is arranged on the optical axis of the optical path of the illumination light, the illumination light can enter into the eye from positions eccentric from the pupil center (specifically, point-symmetrical positions centered on the pupil center).

Further, the light amount distribution of the light passing through the aperture(s) formed in the iris aperture 12 can be changed by changing a relative position between the illumination light source 11 and the aperture(s) formed in the iris aperture 12.

The slit 13 (specifically, aperture(s) described below) can be arranged at the fundus conjugate position P. The aperture formed in the slit 13 defines a shape of an irradiated region (irradiated pattern shape) of the illumination light on the fundus Ef of the eye E to be examined.

The slit 13 can be moved in an optical axis direction of the slit projection optical system 10 using a movement mechanism (not shown). The movement mechanism moves the slit 13 in the optical axis direction, under the control from a controller described below. This allows to move the position of the slit 13 in accordance with the state (specifically, the dioptric power or the shape of the fundus Ef) of the eye E to be examined.

In some embodiments, the slit 13 is configured so that at least one of the position of the aperture or the shape of the aperture can be changed in accordance with the state of the eye E to be examined without being moved in the optical axis direction. The function of the slit 13 with these functions is, for example, realized by a liquid crystal shutter.

The light from the illumination light source 11 that has passed through the aperture(s) formed in the iris aperture 12 passes through the aperture(s) formed in the slit 13, is transmitted through the projection lens 14, and is output as the slit-shaped illumination light. The slit-shaped illumination light output from slit projection optical system 10 is guided to the hole mirror 30.

In some embodiments, the slit projection optical system 10 includes a projector with a light source, and the projector outputs the slit-shaped illumination light. Examples of the projector include an LCD (Liquid Crystal Display) type projector using a transmissive LCD panel, an LCOS (Liquid Crystal On Silicon) type projector using a reflective LCD panel, a DLP (Digital Light Processing) (registered trademark) type projector using a DMD (Digital Mirror Device).

### (Hole mirror 30)

The hole mirror 30 (specifically, deflection surface described below) can be arranged at the pupil conjugate position Q. The hole mirror 30 has a deflection surface whose orientation (deflection direction) can be changed and functions as a uniaxial optical scanner that guides the illumination light from the slit projection optical system 10 to a reflective surface of the first ellipsoidal mirror 40 described below. The hole is formed in the deflection surface so that the optical axis of the slit light receiving optical system 20 described below passes through it. In other words, the hole mirror 30 has a configuration that allows the returning light of the illumination light to be transmitted through (pass through) the central part and to reflect the illumination light in a peripheral part of the central part.

The hole mirror 30 deflects the illumination light by changing the orientation of the deflection surface so that the irradiated region moves sequentially in a direction (direction of the slit width, shorter direction of the irradiated region) orthogonal to a slit direction (direction in which the slit extends, longitudinal direction of the irradiated region) of the irradiated region at an irradiated site of the illumination light on the eye E to be examined. The hole mirror 30 is configured to be capable of changing the deflection direction of the illumination light, under the control from the controller described below.

The illumination light from the slit projection optical system 10 is deflected on the deflection surface around the hole and is guided to the reflective surface of the first ellipsoidal mirror 40. The returning light of the illumination light from the eye E to be examined passes through the hole formed in the hole mirror 30 via the reflective surface of the first ellipsoidal mirror 40 and is guided to the slit light receiving optical system 20.

In some embodiments, the hole mirror 30 has a function that guides the illumination light from the slit projection optical system 10 to a reflective surface of the first ellipsoidal mirror 40, as a biaxial optical scanner.

In some embodiments, the hole mirror 30 is configured to transmit the wavelength component (or polarization component) of the returning light of the illumination light. In this case, the returning light of the illumination light from the eye E to be examined is transmitted through the hole mirror 30 via the reflective surface of the first ellipsoidal mirror 40, and is guided to the slit light receiving optical system 20.

### (Slit light receiving optical system 20)

The slit light receiving optical system 20 is configured to receive the returning light of the illumination light from the eye E to be examined that has passed through the hole of the hole mirror 30. The slit light receiving optical system 20 includes an image sensor 21 and an imaging lens 22.

The image sensor 21 realizes the function of the two-dimensional image sensor as a pixelated photodetector. The light receiving surface (detecting surface, imaging surface) of the image sensor 21 can be arranged at the fundus conjugate position P. The image sensor 21 is configured to be set a light receivable range (virtual opening range, focal plane) that can be moved at the fundus conjugate position P.

For example, the light receiving result(s) acquired by the image sensor 21 is/are read out using a rolling shutter method. In some embodiments, the controller described below performs readout control of the light receiving result(s) by controlling the image sensor 21. In some embodiments, the image sensor 21 can automatically output the light receiving result(s) for a predetermined number of lines, along with information indicating the light receiving position(s).

The image sensor 21 with such a configuration includes a CMOS (Complementary metal oxide semiconductor) image sensor. In this case, the image sensor 21 includes a plurality of pixels (light receiving elements). The plurality of pixels includes a plurality of pixel groups arranged in a column direction. Each of the plurality of pixel groups includes pixels arranged in a row direction. Specifically, the image sensor 21 includes a plurality of pixels arranged two-dimensionally, a plurality of vertical signal lines, and a horizontal signal line. Each pixel includes a photodiode (light receiving element), and a capacitor. The vertical signal lines are provided for each pixel group in the column direction (vertical direction) orthogonal to the row direction (horizontal direction). Each of the vertical signal lines is selectively electrically connected to the pixel group in which the electrical charge corresponding to the light receiving result is accumulated. The horizontal signal line is selectively electrically connected to the vertical signal lines. Each of the pixels accumulates the electrical charge corresponding to the light receiving result of the returning light. The accumulated electrical charge is read out sequentially for each pixel group in the row direction, for example. For example, for each line in the row direction, a voltage corresponding to the electrical charge accumulated in each pixel is supplied to the vertical signal line. The vertical signal lines are selectively electrically connected to the horizontal signal line. By performing readout operation for each line in the row direction described above sequentially in the vertical direction, the light receiving results of the plurality of pixels arranged two-dimensionally can be read out.

By capturing (reading out) the light receiving results of the returning light using the rolling shutter method for this type of image sensor 21, the light receiving image corresponding to a desired virtual opening (aperture) shape extending in the row direction is acquired. Such control is disclosed in, for example, U.S. Patent No. 7831106, U.S. Patent No. 8237835, or the like.

The imaging lens 22 forms an image of the returning light of the illumination light that has passed through the hole formed in the hole mirror 30 (or returning light of the illumination light that has been transmitted through the hole mirror 30) on the light receiving surface of the image sensor 21.

### (First ellipsoidal mirror 40)

The reflective surface (first reflective surface) of the first ellipsoidal mirror 40 is an elliptical surface (specifically, a part of the elliptical surface). The first ellipsoidal mirror 40 is an example of the concave mirror.

The first ellipsoidal mirror 40 has two optically conjugate focal points (first focal point F1, second focal point F2). The hole mirror 30 (deflection surface of the hole mirror 30) is positioned at the first focal point F1 of the first ellipsoidal mirror 40 or near the first focal point F1. In some embodiments, the hole mirror 30 is positioned at a position conjugate optically to the first focal point F1 (conjugate position of the first focal point F1) or near the position conjugate optically to the first focal point F1.

### (Second ellipsoidal mirror 50)

The reflective surface (second reflective surface) of the second ellipsoidal mirror 50 is an elliptical surface (specifically, a part of the elliptical surface). The second ellipsoidal mirror 50 is an example of the concave mirror.

The second ellipsoidal mirror 50 has two optically conjugate focal points (first focal point F3, second focal point F4). The second ellipsoidal mirror 50 is arranged so that the first focal point F3 substantially coincides with the second focal point F2 of the first ellipsoidal mirror 40. In some embodiments, the second ellipsoidal mirror 50 is arranged so that the first focal point F3 substantially coincides with a position conjugate optically to the second focal point F2 of the first ellipsoidal mirror 40 (conjugate position of the second focal point F2) or near the position conjugate optically to the second focal point F2. The eye E to be examined is arranged at the second focal point F4 of the second ellipsoidal mirror 50. In other words, the second ellipsoidal mirror 50 is arranged so that the second focal point F4 substantially coincides with eye to be examined position where the eye E to be examined is arranged.

Thus, a scanning optical member does not need to be disposed at the second focal point F2 (first focal point F3) between the first ellipsoidal mirror 40 and the second ellipsoidal mirror 50. Therefore, the scan range in a predetermined scan direction (lateral direction, horizontal direction) is not limited. For example, in the configuration described in Patent Document 1, a deflecting member is provided for scanning the illumination light in the lateral direction, which theoretically allows an angle of view for imaging of up to 180 degrees (realistically, up to about 150 degrees). In contrast, according to the embodiments, the deflecting member for scanning in the lateral direction does not need to be disposed. Therefore, imaging up to an angle of view beyond 180 degrees becomes possible (since the cornea of the human eye is positioned at a position projected forward from the pupil, it is possible to observe a range beyond 180 degrees with a fisheye lens-like effect).

The first ellipsoidal mirror 40 and the second ellipsoidal mirror 50 are an example of the "two concave mirrors" according to the embodiments. By holding the first ellipsoidal mirror 40 and the second ellipsoidal mirror 50 with the holding member as follows, the first ellipsoidal mirror 40 and the second ellipsoidal mirror 50 can be arranged easily and at low cost, so as to coincide with the second focal point F2 of the first ellipsoidal mirror 40 and the first focal point F3 of the second ellipsoidal mirror 50 with high precision.

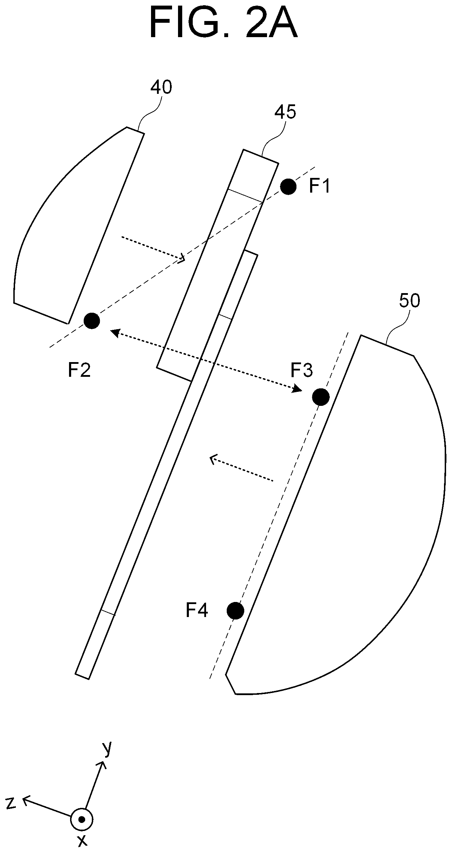

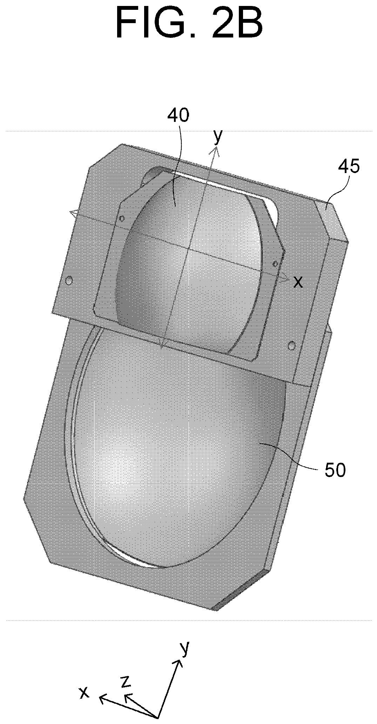

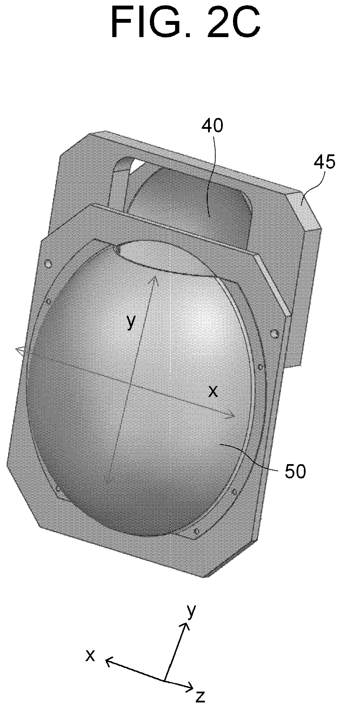

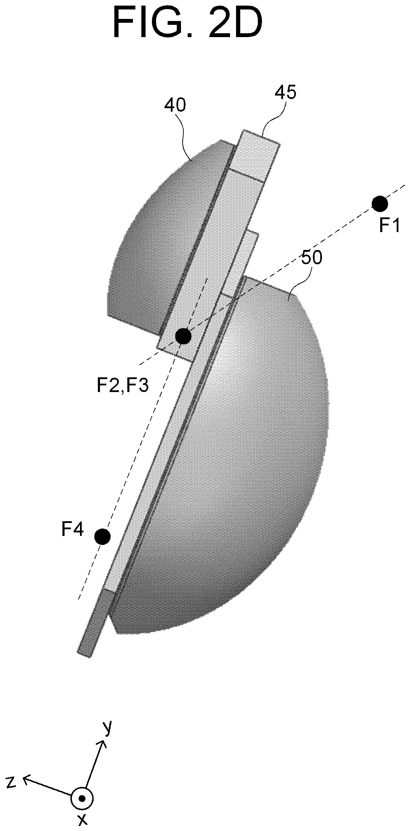

FIG. 2A, FIG. 2B, FIG. 2C, and FIG. 2D schematically show examples of a holding structure of the first ellipsoidal mirror 40 and the second ellipsoidal mirror 50. FIG. 2A is a schematic side view illustrating a mode of an installation of the first ellipsoidal mirror 40 and the second ellipsoidal mirror 50 held by the holding member 45. FIG. 2B is a perspective view schematically illustrating a state in which the first ellipsoidal mirror 40 and the second ellipsoidal mirror 50 are held by the holding member 45, when viewed from the side of the reflective surface of the second ellipsoidal mirror 50. FIG. 2C is a perspective view schematically illustrating a state in which the first ellipsoidal mirror 40 and the second ellipsoidal mirror 50 are held by the holding member 45, when viewed from the side of the reflective surface of the first ellipsoidal mirror 40. FIG. 2D is a side view schematically illustrating a state in which the first ellipsoidal mirror 40 and the second ellipsoidal mirror 50 are held by the holding member 45. In FIG. 2A, FIG. 2B, FIG. 2C, and FIG. 2D, like reference numerals designate like parts as in FIG. 1. The same description may not be repeated.

The fundus observation apparatus 1 includes the holding member 45 configured to hold the first ellipsoidal mirror 40 and the second ellipsoidal mirror 50. The holding member 45 can fix the first ellipsoidal mirror 40 and the second ellipsoidal mirror 50 in a state where a relative position of the first ellipsoidal mirror 40 and the second ellipsoidal mirror 50 are arranged so as to be in a default positional relationship in the optical axis direction of the optical system including the slit projection optical system 10 and the slit light receiving optical system 20.

Flanges are formed on the periphery of the first ellipsoidal mirror 40 and the second ellipsoidal mirrors 50, respectively. The first ellipsoidal mirror 40 can be fixed to the holding member 45 by fixing the flange of the first ellipsoidal mirror 40 to the holding member 45. The second ellipsoidal mirror 50 can be fixed to the holding member 45 by fixing the flange of the second ellipsoidal mirror 50 to the holding member 45.

The first ellipsoidal mirror 40 is formed, for example, using an electroforming process. The electroforming process is a method of faithfully replicating the shape of the matrix at the nanolevel by electrochemically depositing metal ions in an electrolytic solution on the surface of the matrix. This allows to form the first ellipsoidal mirror 40 having a very high precision shape.

Both of the first focal point F1 and the second focal point F2 of the first ellipsoidal mirror 40 are positioned farther away from the surface of the flange relative to the concave reflective surface. In FIG. 2A, both of a distance in a direction of the minor axis between the reflective surface and the first focal point F 1, and a distance in the direction of the minor axis between the reflective surface and the second focal point F2 are longer than a distance in the direction of the minor axis between the reflective surface and the surface of the flange.

Both ends in a direction of the major axis of the first ellipsoidal mirror 40 (predetermined first direction) have shape cut in a plane orthogonal to the direction of the major axis. This allows to reduce the weight and size of the first ellipsoidal mirror 40 while securing the size of the reflective surface of the first ellipsoidal mirror 40 necessary for wide-angle fundus observation.

The second ellipsoidal mirror 50 is formed, for example, using the electroforming process, in the same way as the first ellipsoidal mirror 40. This allows to form the second ellipsoidal mirror 50 having a very high precision shape.

Both of the first focal point F3 and the second focal point F4 of the second ellipsoidal mirror 50 are positioned farther away from the surface of the flange relative to the concave reflective surface. In FIG. 2A, both of a distance in a direction of the minor axis between the reflective surface and the first focal point F3, and a distance in the direction of the minor axis between the reflective surface and the second focal point F4 are longer than a distance in the direction of the minor axis between the reflective surface and the surface of the flange. It should be noted that the flange (surface) formed on the second ellipsoidal mirror 50 is approximately parallel to the major axis connecting the first focal point F3 and the second focal point F4.

Both ends in a direction of the major axis of the second ellipsoidal mirror 50 (predetermined first direction) have shape cut in a plane intersecting the direction of the major axis. This allows to reduce the weight and size of the second ellipsoidal mirror 50 while securing the size of the reflective surface of the second ellipsoidal mirror 50 necessary for wide-angle fundus observation. In particular, by cutting the lower side, the interference between the mouth and jaw of the examinee to be observed and the second ellipsoidal mirror 50 can be avoided.

The holding member 45 has a predetermined thickness in the optical axis direction of the optical system. An opening is formed in the holding member 45. The opening is formed so that the reflective surface of the first ellipsoidal mirror 40 held on a first holding surface is exposed on the side of a second holding surface opposite the first holding surface, and that the reflective surface of the second ellipsoidal mirror 50 held on a second holding surface is exposed on the side of the first holding surface. As a result, the light from the optical system reflected on the reflective surface of the first ellipsoidal mirror 40 passes through the opening, is reflected on the reflective surface of the second ellipsoidal mirror 50, and is guided to the fundus. While the returning light from the fundus reflected on the reflective surface of the second ellipsoidal mirror 50 passes through the opening, is reflected on the reflective surface of the first ellipsoidal mirror 40, and is guided to the optical system.

It should be noted the thickness of the holding member 45 in the optical axis direction of the optical system does not have to be constant. According to the change in thickness of the optical axis direction of the 45 optical system of the holding member 45, the peripheries of the first ellipsoidal mirror 40 and the second ellipsoidal mirror 50 should be cut, and the peripheries (flanges) of the ellipsoidal mirrors should have shapes corresponding to the change in thickness.

As shown in FIG. 2A, FIG. 2B, and FIG. 2C, the first ellipsoidal mirror 40 is arranged so that the reflective surface faces the opening at the first holding surface of the holding member 45, and the second ellipsoidal mirror 50 is arranged so that the reflective surface faces the opening at the second holding surface. When the first ellipsoidal mirror 40 and the second ellipsoidal mirror 50 are placed on the holding member 45 in this manner, the second focal point F2 of the first ellipsoidal mirror 40 and the first focal point F3 of the second ellipsoidal mirror 50 are substantially coincident in the optical axis direction of the optical system, as shown in FIG. 2D.

Therefore, as shown in FIG. 2B and FIG. 2C, the second focal point F2 of the first ellipsoidal mirror 40 and the first focal point F3 of the second ellipsoidal mirror 50 can be easily and precisely matched in three dimensions, by merely changing the relative position of the first ellipsoidal mirror 40 and the second ellipsoidal mirror 50 in two-dimensional directions (x and y directions) orthogonal to the optical axis direction of the optical system.

For example, in a state where the first ellipsoidal mirror 40 and the second ellipsoidal mirror 50 are placed on the both sides of the holding member 45 as shown in FIG. 2D, the light from the light source positioned at the first focal point F1 of the first ellipsoidal mirror 40 or the position substantially conjugate optically to the first focal point F1 is received at the second focal point F4 of the second ellipsoidal mirror 50 or the position substantially conjugate optically to the second focal point F4. In this case, by changing the relative position of the first ellipsoidal mirror 40 and the second ellipsoidal mirror 50 in the two-dimensional directions (x and y directions) orthogonal to the optical axis direction of the optical system, so that the light from the light source is focused at the second focal point F2 of the first ellipsoidal mirror 40, the first focal point F3 of the second ellipsoidal mirror 50, and the second focal point F4 of the second ellipsoidal mirror 50, the relative position of the first ellipsoidal mirror 40 and the second ellipsoidal mirror 50 in the two-dimensional directions (x and y directions) orthogonal to the optical axis direction is determined.

In some embodiments, the fundus observation apparatus 1 includes a first movement mechanism and a second movement mechanism. The first movement mechanism is configured to hold the first ellipsoidal mirror 40 and to change a relative position of the first ellipsoidal mirror 40 relative to the holding member 45 in the two-dimensional directions (x and y directions) orthogonal to the optical axis direction of the optical system. The second movement mechanism is configured to hold the second ellipsoidal mirror 50 and to change a relative position of the second ellipsoidal mirror 50 relative to the holding member 45 in the two-dimensional directions (x and y directions) orthogonal to the optical axis direction of the optical system. In this case, the fundus observation apparatus 1 is configured to change the relative position of the first ellipsoidal mirror 40 and the second ellipsoidal mirror 50 using at least one of the first movement mechanism or the second movement mechanism.

As described above, once the relative position of the first ellipsoidal mirror 40 and the second ellipsoidal mirror 50 is determined, the flange of each ellipsoidal mirror is fixed to the holding member 45. For example, the flange is fixed to the holding member 45 with screwing (screw clamp), pinning, crimping, welding, caulking, or the like.

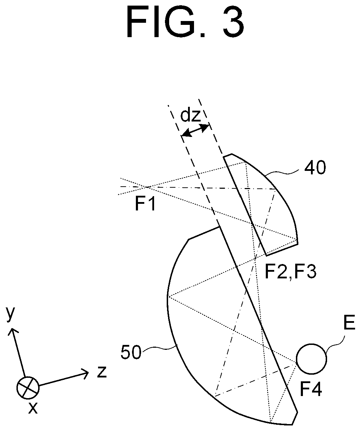

FIG. 3 schematically shows a side view of the first ellipsoidal mirror 40 and the second ellipsoidal mirror 50 held by the holding member 45. In FIG. 3, parts similar to those in FIGs. 1 to 2D are denoted by the same reference symbols, and description thereof is omitted as appropriate.

As described above, the holding member 45 is configured to hold the flange of the first ellipsoidal mirror 40 and the flange of the second ellipsoidal mirror 50. Here, as shown in FIG. 2D, the holding member 45 is configured to hold the flange (surface) of the first ellipsoidal mirror 40 and the flange (surface) of the second ellipsoidal mirror 50 so that flange of the first ellipsoidal mirror 40 and the flange of the second ellipsoidal mirror 50 are approximately parallel to each other.

This allows to perform position matching on the first ellipsoidal mirror 40 and the second ellipsoidal mirror 50 in the x and y directions orthogonal to the z direction with high precision, in a state where the first ellipsoidal mirror 40 and the second ellipsoidal mirror 50 have been performed position matching in the z direction with high precision with the distance dz in the z direction held constant by the holding member 45. As a result, the first ellipsoidal mirror 40 and the second ellipsoidal mirror 50 can be performed position matching easily, at low cost, and with high precision.

Further, the both ends of the second ellipsoidal mirror 50 according to the embodiments have shape cut in a plane intersecting the direction of the major axis of the reflective surface that is a part of the elliptical surface, as described above. By cutting the both ends, weight and size can be reduced. However, in particular, by cutting the lower end, the burden on the examinee to be observed can be reduced.

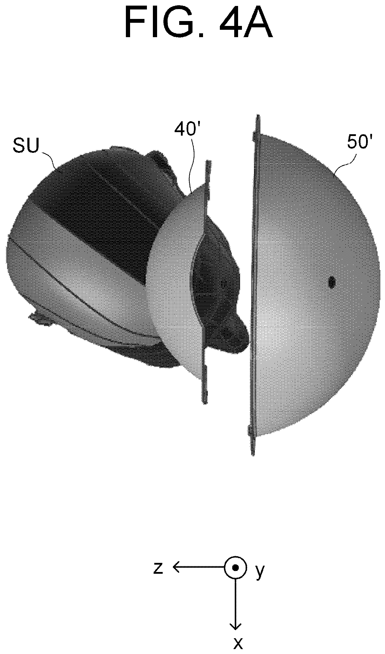

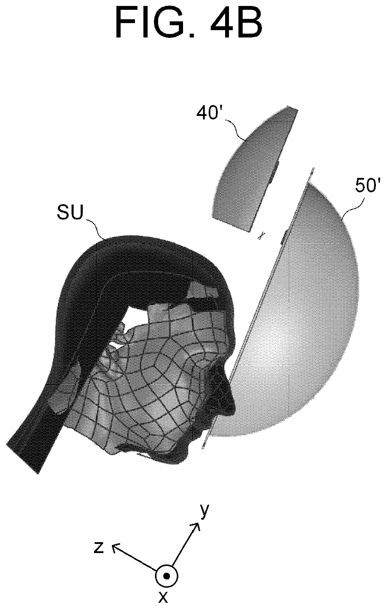

FIG. 4A and FIG. 4B schematically show a positional relationship between the first and second ellipsoidal mirrors according to a comparative example of the embodiments and the examinee to be observed. FIG. 4A is a top view illustrating a broad outline of a positional relationship between the first and second ellipsoidal mirrors according to the comparative example of the embodiments and the examinee to be observed. FIG. 4B is a side view illustrating a broad outline of a positional relationship between the first and second ellipsoidal mirrors according to the comparative example of the embodiments and the examinee to be observed.

The fundus observation apparatus according to the comparative example of the embodiments includes a first ellipsoidal mirror 40' and a second ellipsoidal mirror 50'. The first ellipsoidal mirror 40' may be the same as the first ellipsoidal mirror 40 according to the embodiments. In contrast, the both ends of the second ellipsoidal mirror 50' are not cut in a plane intersecting direction of the major axis of the reflective surface that is a part of the elliptical surface (see FIG. 4B).

In this case, when the examinee SU moves his/her face close to the second ellipsoidal mirror 50' in order to place the eye to be examined at the eye to be examined position (the position of the second focal point F4 of the second ellipsoidal mirror 50'), a part of the face (mouth, chin) interferes with the lower part of the second ellipsoidal mirror 50' as shown in FIG. 4B. Therefore, the examinee SU will need to place the eye to be examined at the eye to be examined position with the face facing oblique to the reflective surface of the second ellipsoidal mirror 50', as shown in FIG. 4A. Thereby, it may cause difficulty in visual fixation of the eye to be examined or may burden the posture of the examinee SU during observation.

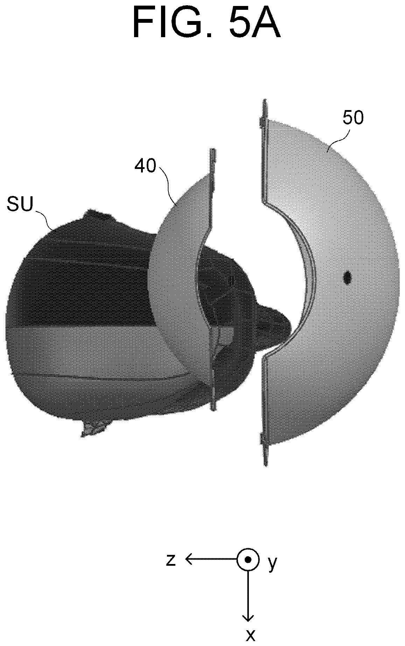

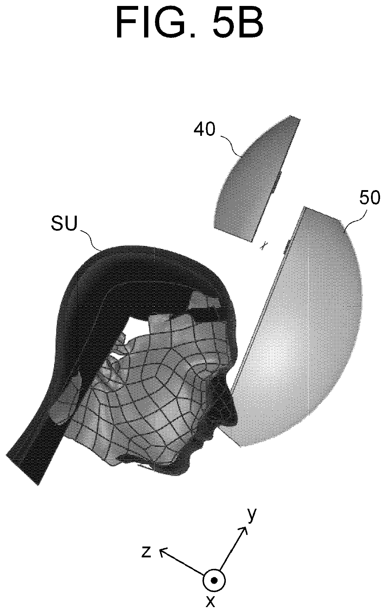

FIG. 5A and FIG. 5B schematically show a positional relationship between the first and second ellipsoidal mirrors according to the embodiments and the examinee to be observed. FIG. 5A is a top view illustrating a broad outline of a positional relationship between the first and second ellipsoidal mirrors according to the embodiments and the examinee to be observed. FIG. 5B is a side view illustrating a broad outline of a positional relationship between the first and second ellipsoidal mirrors according to the embodiments and the examinee to be observed.

As described above, the both ends of the second ellipsoidal mirror 50 are cut in a plane intersecting direction of the major axis of the reflective surface that is a part of the elliptical surface (see FIG. 5B). In this case, even if the examinee SU moves his/her face close to the second ellipsoidal mirror 50 in order to place the eye to be examined at the eye to be examined position (the position of the second focal point F4 of the second ellipsoidal mirror 50), the face (mouth, chin) can avoid interfering with the lower part of the second ellipsoidal mirror 50, as shown in FIG. 5B. Therefore, the examinee SU can place the eye to be examined at the eye to be examined position while facing front to the reflective surface of the second ellipsoidal mirror 50, as shown in FIG. 5A. Thereby, this facilitates the visual fixation of the eye to be examined, and does not burden the posture of the examinee SU during observation.

It should be noted that, as shown in FIG. 1, the second ellipsoidal mirror 50 is arranged so that an angle between a straight line connecting the first focal point F1 and the second focal point F2 of the first ellipsoidal mirror 40 and a straight line connecting the first focal point F3 and the second focal point F4 of the second ellipsoidal mirror 50 is an angle α. For example, the angle α is 30 degrees. In some embodiments, the second ellipsoidal mirror 50 is configured to be movable relative to the first ellipsoidal mirror 40 so as to change the angle α.

In such a configuration, the illumination light deflected by the hole mirror 30 arranged at the first focal point F1 is reflected on the reflective surface of the first ellipsoidal mirror 40 and is guided to the second focal point F2 of the first ellipsoidal mirror 40. The illumination light that has been guided to the second focal point F2 is guided to the reflective surface of the second ellipsoidal mirror 50, is reflected on this reflective surface, and is guided to the eye E to be examined arranged at the second focal point F4 of the second ellipsoidal mirror 50.

The illumination light that has been guided to the eye E to be examined enters the eye through the pupil and is irradiated onto the fundus Ef. The returning light of the illumination light reflected on the fundus Ef is emitted outside of the eye E to be examined through the pupil, travels in the same path as the outward path in the opposite direction, and is guided to the first focal point F1 of the first ellipsoidal mirror 40. The returning light of the illumination light that has been guided to the first focal point F1 passes through the hole formed in the hole mirror 30 (or is transmitted through the hole mirror 30), and is guided to the slit light receiving optical system 20, as described above.

In some embodiments, at least one of the first ellipsoidal mirror 40 or the second ellipsoidal mirror 50 is a concave mirror whose reflective surface is formed in a concave shape. In some embodiments, the reflective surface of the concave mirror is formed to be a free-form surface.

In addition to the configuration shown in FIG. 1, the fundus observation apparatus 1 may be provided with an alignment optical system for performing position matching between the eye E to be examined and the optical system. The fundus observation apparatus 1 may be provided with a focusing mechanism using the movement of the lens or the movement of the slit light receiving optical system 20.

Further, the fundus observation apparatus 1 may have a configuration for providing a function associated with the inspection. For example, the fundus observation apparatus 1 may be provided with a fixation optical system for projecting a visual target (fixation target) for fixating the eye E to be examined onto the fundus Ef of the eye E to be examined. Further, the fundus observation apparatus 1 may be provided with any element or unit, such as a member for supporting the face of the examinee (chin rest, forehead pad, etc.).

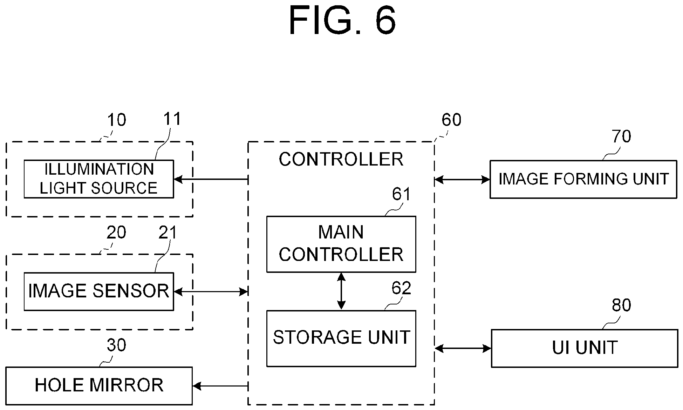

FIG. 6 shows an example of a configuration of a processing system of the fundus observation apparatus 1 according to the first embodiment. In FIG. 6, like parts are designated by like reference numerals as in FIG. 1 and repetitious description of such parts may not be provided.

The processing system of the fundus observation apparatus 1 is configured with a controller 60 as a center. The controller 60 controls each part of the fundus observation apparatus 1.

The controller 60 includes a main controller 61 and a storage unit 62. The functions of the main controller 61 are realized by a processor, for example. The storage unit 62 stores, in advance, computer programs for controlling the fundus observation apparatus 1. Examples of the computer programs include an illumination light source control program, an image sensor control program, a hole mirror control program, an image forming program, and a program for user interface. The main controller 61 operates according to such the computer program(s), and thereby the controller 60 performs the control processing.

### (Main controller 61)

The main controller 61 controls each of the slit projection optical system 10, the slit light receiving optical system 20, the hole mirror 30, the image forming unit 70, and a user interface (UI) unit 80.

Examples of the control for the slit projection optical system 10 include control for the illumination light source 11. Examples of the control for the illumination light source 11 include turning the light source on and off, adjustment of the light amount, and adjustment of an aperture.

Examples of the control for the slit light receiving optical system 20 include control for the image sensor 21. Examples of the control for the image sensor 21 include control of setting the light receivable range (virtual opening range, focal plane) that can be moved at the fundus conjugate position P and control for reading out the light receiving result(s) using a rolling shutter method (for example, setting of light receiving size corresponding to the size of the illumination pattern, or the like). Further, the control for the image sensor 21 includes the reset control, the exposure control, the charge transfer control, and the output control.

Examples of the control for the hole mirror 30 include control of the angle of the deflection surface for deflecting the illumination light. By controlling the angle of the deflection surface, the deflection direction of illumination light can be controlled. By controlling an angle range of the deflection surface, the scan range (scan start position and scan end position) can be controlled. By controlling the speed of changing the angle of deflection surface, the scan speed can be controlled.

Examples of the control for the image forming unit 70 include image forming control for forming images of the eye E to be examined from the light receiving result(s) obtained using the image sensor 21.

Examples of the control for the UI unit 80 include a control for a display device and a control for an operation device (input device).

### (Storage unit 62)

The storage unit 62 stores various types of data. Examples of the data stored in the storage unit 62 include light receiving result(s) obtained by the image sensor 21, image data of an image formed by the image forming unit 70, and information on eye to be examined. The information on eye to be examined includes information on the examinee such as patient ID and name, and information on the eye to be examined such as identification information of the left eye / right eye.

The storage unit 62 further stores various types of programs and data to run the fundus observation apparatus 1.

### (Image forming unit 70)

The image forming unit 70 can form the light receiving image (fundus image) corresponding to an arbitrary light receivable range (virtual opening range, focal plane) based on the light receiving result(s) read out from the image sensor 21 using the rolling shutter method. The image forming unit 70 can sequentially form light receiving light images corresponding to the light receivable ranges (opening ranges) and form an image of the eye E to be examined from a plurality of formed light receiving images. The various images (the various image data) formed by the image forming unit 70 are stored in the storage unit 62, for example.

For example, the image forming unit 70 includes a processor, and executes the function described above by performing processing corresponding to the program(s) stored in the storage unit or the like.

### (UI unit 80)

The UI unit 80 has a function for exchanging information between a user and the fundus observation apparatus 1. The UI unit 80 includes the display device and the operation device. The display device may include a display unit, and it may include another display device. The display device displays various information. The display device includes a liquid crystal display, for example. The display device displays the above information under the control of the main controller 61. Examples of the information displayed on the display device include information corresponding to the control result by the controller 60, information (image) corresponding to the calculation result by the image forming unit 70. The operation device includes various hardware keys and/or various software keys. Upon receiving the operation content for the operation device, the main controller 61 can output a control signal corresponding to the operation content to each part of the fundus observation apparatus. At least a part of the display device and at least a part of the operation device may be configured integrally. One example of this is the touch panel display.

The first ellipsoidal mirror 40 is an example of the "first concave mirror" according to the embodiments. The second ellipsoidal mirror 50 is an example the "second concave mirror" according to the embodiments. The slit projection optical system 10 and the slit light receiving optical system 20 are an example of the "optical system" according to the embodiments. The hole mirror 30 is an example of the "deflecting member" according to the embodiments.

### <Operation>

Next, the operation of the fundus observation apparatus 1 according to the first embodiment will be described.

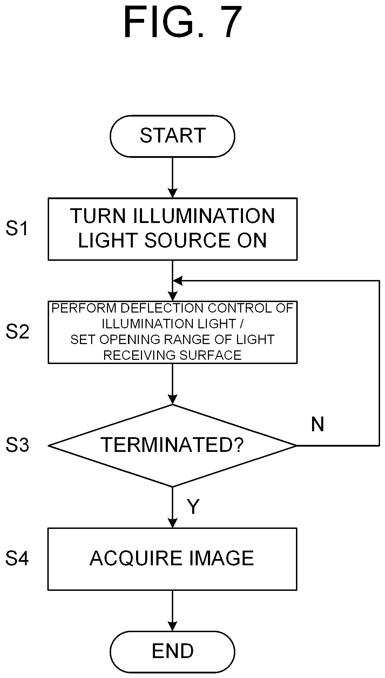

FIG. 7 shows an example of the operation of the fundus observation apparatus 1 according to the first embodiment. FIG. 7 represents a flowchart of an example of the operation of the fundus observation apparatus 1 according to the first embodiment. The storage unit 62 stores a computer program for realizing the processing shown in FIG. 7. The main controller 61 operates according to the computer program, and thereby the main controller 61 performs the processing shown in FIG. 7.

It is assumed that the eye E to be examined is arranged at a predetermined eye to be examined position (the second focal point F4 of the second ellipsoidal mirror 50 in FIG. 1), in FIG. 7.

### (S1: Turn illumination light source on)

The main controller 61 controls the illumination light source 11 to turn the illumination light source 11 on.

The light output from the illumination light source 11 passes through the aperture(s) formed in the iris aperture 12, passes through the aperture(s) formed in the slit 13, is transmitted through the projection lens 14, and is guided to the hole mirror 30 as the slit-shaped illumination light.

### (S2: Deflection control of illumination light and set opening range of light receiving surface)

Subsequently, the main controller 61 controls the hole mirror 30 to set the orientation of the deflection surface in a predetermined deflection direction in order to illuminate a predetermined irradiated range, and starts deflection control of the illumination light that sequentially changes the orientation of the deflection surface within a predetermined deflection angle range. In other words, the main controller 61 starts scanning the illumination light onto the fundus Ef.

In some embodiments, the main controller 61 controls the hole mirror 30 to perform deflection control of the illumination light in synchronization with the movement of the virtual opening range (light receivable range) that can be arbitrarily set in the image sensor 21.

In some embodiments, the main controller 61 controls the image sensor 21 to virtually set the opening range (light receivable range) including a light receiving range of the returning light on the light receiving surface corresponding to the irradiated region of the illumination light on the fundus Ef. For example, the irradiated range of the illumination light on the fundus Ef can be identified based on the deflection angle of the deflection surface of the hole mirror 30. The main controller 61 can virtually set the opening range on the light receiving surface of the image sensor 21 corresponding to the deflection direction of the deflection surface of the hole mirror 30 that is changed sequentially.

The illumination light guided to the hole mirror 30 is deflected on the deflection surface whose deflection direction has been changed to guide to the reflective surface of the first ellipsoidal mirror 40, is reflected on this reflective surface, and is guided to the reflective surface of the second ellipsoidal mirror 50 via the second focal point F2 of the first ellipsoidal mirror 40. The illumination light guided to the reflective surface of the second ellipsoidal mirror 50 is reflected on this reflective surface, enters the eye of the eye E to be examined arranged at the second focal point F2 of the second ellipsoidal mirror 50, and is irradiated onto the fundus Ef. The returning light of the illumination light from the fundus Ef travels in the same path as the outward path in the opposite direction, passes through the hole formed in the hole mirror 30 or is transmitted through the hole mirror 30, and is received on the light receiving surface of the image sensor 21 through the imaging lens 22. On the light receiving surface of image sensor 21, the virtual opening range (light receivable range) is set so as to include the light receiving range of the returning light corresponding to the irradiated range of the illumination light on the fundus Ef. Thereby, the returning light alone from fundus Ef can be received while suppressing the effect of unnecessary scattered light.

### (S3: Terminated?)

Subsequently, the main controller 61 determines whether or not to terminate the scanning of the illumination light for the fundus Ef. For example, the main controller 61 can determine whether or not to terminate the scanning of the illumination light for the fundus Ef, by determining whether or not the deflection angle of the deflection surface of the hole mirror 30, which is changed sequentially, is within the predetermined deflection angle range.

When it is determined to terminate the scanning of the illumination light for the fundus Ef (S3: Y), the operation of the fundus observation apparatus 1 proceeds to step S4. When it is determined not to terminate the scanning of the illumination light for the fundus Ef (S3: N), the operation of the fundus observation apparatus 1 proceeds to step S2.

### (S4: Acquire image)

When it is determined in step S3 to terminate the scanning of the illumination light for the fundus Ef (S3: Y), the main controller 61 controls the image forming unit 70 to form an image of the eye E to be examined based on the light receiving result(s) read out from the image sensor 21. In some embodiments, the image forming unit 70 sequentially forms the light receiving images based on the light receiving results read from the image sensor 21 in step S2, and forms an image of the eye E to be examined from the plurality of formed light receiving images.

This terminates the operation of the fundus observation apparatus 1 (END).

As explained above, according to the first embodiment, the position matching between the first ellipsoidal mirror 40 and the second ellipsoidal mirror 50 can be performed easily, at low cost, and with high precision. Furthermore, in the fundus observation apparatus 1 equipped with such the first ellipsoidal mirror 40 and the second ellipsoidal mirror 50, the optical path of the slit projection optical system 10 and the optical path of the slit light receiving optical system 20 are combined using the hole mirror 30, and the illumination light is deflected and is guided to the reflective surface of the first ellipsoidal mirror 40 using the hole mirror 30. Thereby, a shared optical system shared between the optical path of the wide-angle illumination light and the optical path of the returning light can be easily arranged while ensuring an angle of view for imaging of more than 80 degrees with the optical system alone that scans in a slit width direction (width direction of a line) perpendicular to the slit direction of the illumination light, with a low cost and simple configuration. Further, the optical system can also be arranged on the passing (transmitting) side of the hole in the hole mirror 30. Therefore, the configuration does not require a pupil relay system, thereby increasing the degree of freedom in the arrangement of the optical system.

Furthermore, the deflection angle range can be widened while reducing noise, by using the hole mirror 30 instead of a polygon mirror or the like. In addition, the scan range of the illumination light can be set as desired, by varying the length in the slit direction or the length in the scan direction.

### <Second embodiment>

The configuration of the fundus observation apparatus according to the embodiments is not limited to the configuration of the fundus observation apparatus 1 according to the first embodiment. For example, the fundus observation apparatus 1 according to the first embodiment may further be provided with an OCT optical system.

Hereinafter, in the embodiments, a case of using the swept source type OCT method in the measurement or the imaging (shooting) using OCT will be described. However, the configuration according to the embodiments can also be applied to a fundus observation apparatus using other type of OCT (for example, spectral domain type OCT).

In the following, the fundus observation apparatus according to a second embodiment will be described focusing on differences from the fundus observation apparatus 1 according to the first embodiment.

### <Configuration>

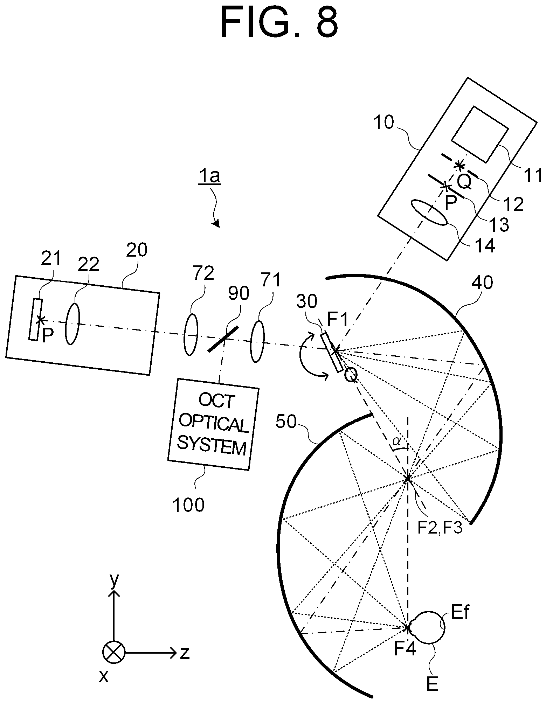

FIG. 8 shows an example of a configuration of an optical system of the fundus observation apparatus according to the second embodiment. In FIG. 8, like parts are designated by like reference numerals as in FIG. 1 and repetitious description of such parts may not be provided.

The configuration of the optical system in the fundus observation apparatus 1a according to the second embodiment differs from that of the optical system in the fundus observation apparatus 1 according to the first embodiment in that an OCT optical system 100 is added to the configuration of the optical system in the fundus observation apparatus 1. The optical path of the OCT optical system 100 is coupled with the optical path of the slit light receiving optical system 20 in the optical path between the slit light receiving optical system 20 and the hole mirror 30.

Specifically, a relay lens optical system including relay lenses 71 and 72 is arranged in the optical path between the slit light receiving optical system 20 and the hole mirror 30. The optical path between the relay lens 71 and the relay lens 72 is converted into an optical path of a telecentric optical system, and a dichroic mirror 90 is arranged in the optical path of the telecentric optical system. In other words, the relay lens optical system converts at least part of the optical path where the dichroic mirror 90 is arranged into the optical path of the telecentric optical system.

The dichroic mirror 90 is an optical path coupling separating member that separates the optical path of the OCT optical system 100 from the optical path of the slit light receiving optical system 20 (or that couples the optical path of the slit light receiving optical system 20 and the optical path of the OCT optical system 100). The dichroic mirror 90 reflects the measurement light from the OCT optical system 100 to guide the measurement light to the relay lens 71, and reflects the returning light of the measurement light from eye E to be examined to guide the returning light to the OCT optical system 100. Further, the dichroic mirror 90 transmits the returning light of the illumination light from the eye E to be examined that has been guided through the relay lens 71 and guides the returning light to the relay lens 72.

### (OCT optical system 100)

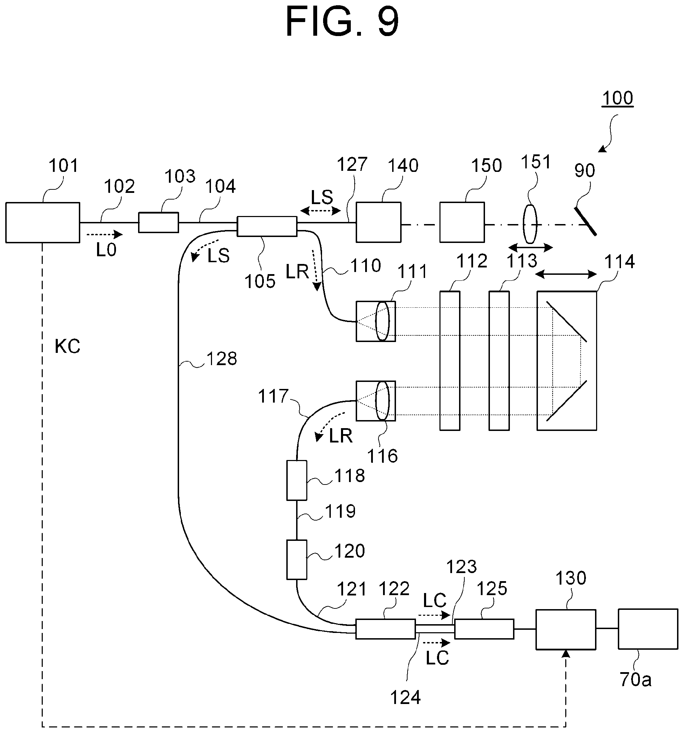

FIG. 9 shows an example of the configuration of the OCT optical system 100 in FIG. 8. In FIG. 9, like parts are designated by like reference numerals as in FIG. 8 and repetitious description of such parts may not be provided.

The OCT optical system 100 is provided with an optical system for performing OCT measurement (or OCT imaging) on the eye E to be examined. This optical system is an interference optical system that splits light from a wavelength sweeping type (i.e., a wavelength scanning type) light source into measurement light and reference light, makes the measurement light returning from the eye E to be examined and the reference light having traveled through the reference optical path interfere with each other to generate interference light, and detects the interference light. The detection result (detection signal) of the interference light obtained by the interference optical system is a signal indicating a spectrum of the interference light, and is sent to the image forming unit 70a, the data processor 75a, and the like which are described later.

Like swept source type fundus observation apparatuses commonly used, the OCT light source 101 includes a wavelength sweeping type (i.e., a wavelength scanning type) light source capable of sweeping (scanning) the wavelengths of emitted light. A laser light source including a resonator and emitting light having a center wavelength of 1050 nm, for example, is used as the wavelength sweeping type light source. The OCT light source 101 temporally changes the output wavelength in the near infrared wavelength band which cannot be visually recognized by the human eye.

Light L0 output from the OCT light source 101 is guided to the polarization controller 103 through the optical fiber 102, and the polarized wave state of the light L0 is adjusted. The polarization controller 103, for example, applies external stress to the looped optical fiber 102 to thereby adjust the polarization state of the light L0 guided through the optical fiber 102.

The light L0 whose the polarization state has been adjusted by the polarization controller 103 is guided to the fiber coupler 105 through the optical fiber 104, and is split into the measurement light LS and the reference light LR.

The reference light LR is guided to the collimator 111 through the optical fiber 110. The reference light LR is converted into a parallel light flux by the collimator 111. Then, the reference light LR is guided to the optical path length changing unit 114 via an optical path length correction member 112 and a dispersion compensation member 113. The optical path length correction member 112 acts so as to match the optical path length of the reference light LR with the optical path length of the measurement light LS. The dispersion compensation member 113 acts so as to match the dispersion characteristics between the reference light LR and the measurement light LS.

The optical path length changing unit 114 is movable in directions indicated by the arrow in FIG. 9, thereby changing the length of the optical path of the reference light LR. Through such movement, the length of the optical path of the reference light LR is changed. The change in the optical path length is used for the correction of the optical path length according to the axial length of the eye E to be examined, for the adjustment of the interference state, or the like. The optical path length changing unit 114 includes, for example, a corner cube and a movement mechanism for moving the corner cube. In this case, the corner cube in the optical path length changing unit 114 changes the traveling direction of the reference light LR that has been made into the parallel light flux by the collimator 111 in the opposite direction. The optical path of the reference light LR incident on the corner cube and the optical path of the reference light LR emitted from the corner cube are parallel.

The reference light LR that has traveled through the optical path length changing unit 114 passes through the dispersion compensation member 113 and the optical path length correction member 112, is converted from the parallel light flux to the convergent light flux by a collimator 116, and enters an optical fiber 117. The reference light LR that has entered the optical fiber 117 is guided to a polarization controller 118, and the polarization state of the reference light LR is adjusted. Then the reference light LR is guided to an attenuator 120 through an optical fiber 119, and the light amount of the reference light LR is adjusted. After that, the reference light LR is guided to a fiber coupler 122 through an optical fiber 121.

Meanwhile, the measurement light LS generated by the fiber coupler 105 is guided through the optical fiber 127, and is made into the parallel light flux by the collimator lens unit 140. The measurement light LS which has been made into the parallel light flux is deflected one-dimensionally or two-dimensionally by an optical scanner 150.

The collimator lens unit 140 includes a collimator lens. The collimator lens is disposed on an optical axis of the interference optical system included in the OCT optical system 100. The collimator lens converts a light flux of the measurement light emitted from the end of an optical fiber into a parallel light flux. Here, the optical fiber is connected to the OCT optical system 100 and guides the measurement light LS to the end. The end of the optical fiber is arranged at the fundus conjugate position P, for example.

The optical scanner 150 (deflection surface) can be arranged at the pupil conjugate position Q. In case of deflecting the illumination light in a one-dimensionally manner, the optical scanner 150 includes a galvano scanner that deflects the measurement light LS within a predetermined deflection angle range with reference to a predetermined deflection direction. In case of deflecting the illumination light in a two-dimensionally manner, the optical scanner 150 includes a first galvano scanner and a second galvano scanner. The first galvano scanner deflects the measurement light LS so as to move the irradiated position in a horizontal direction (for example, x direction) orthogonal to an optical axis of the OCT optical system 100. The second galvano scanner deflects the measurement light LS deflected by the first galvano scanner so as to move the irradiated position in a vertical direction (for example, y direction) orthogonal to the optical axis of the OCT optical system 100. Examples of scan mode for moving the irradiated position of the measurement light LS using the optical scanner 150 include a horizontal scan, a vertical scan, a cross scan, a radial scan, a circle scan, a concentric scan, and a helical (spiral) scan.

The measurement light LS deflected by the optical scanner 150 passes through the focusing lens 151, is reflected by the dichroic mirror 90, passes through the hole in the hole mirror 30, is guided to the reflective surface of the first ellipsoidal mirror 40, and is guided to the eye E to be examined along the same path as the illumination light from the slit projection optical system 10. The focusing lens 151 is movable along the optical path of the measurement light LS (optical axis of the OCT optical system 100). The focusing lens 151 is moved along the optical path of the measurement light LS using a movement mechanism (not shown), under the control from the controller described below.

The measurement light LS reflected on the reflective surface of the second ellipsoidal mirror 50 enters the eye of the eye E to be examined at the second focal point F4 (eye to be examined position) through the pupil. The measurement light LS is scattered (and reflected) at various depth positions of the eye E to be examined. The returning light of the measurement light LS including such backscattered light advances through the same path as the outward path in the opposite direction and is guided to the fiber coupler 105, and then reaches the fiber coupler 122 through the optical fiber 128.

The fiber coupler 122 combines (interferes) the measurement light LS incident through the optical fiber 128 and the reference light LR incident through the optical fiber 121 to generate interference light. The fiber coupler 122 generates a pair of interference light LC by splitting the interference light generated from the measurement light LS and the reference light LR at a predetermined splitting ratio (for example, 1 : 1). The pair of the interference light LC emitted from the fiber coupler 122 is guided to the detector 125 through the optical fibers 123 and 124, respectively.

The detector 125 is, for example, a balanced photodiode that includes a pair of photodetectors for respectively detecting the pair of interference light LC and outputs the difference between the pair of detection results obtained by the pair of photodetectors. The detector 125 sends the detection result(s) (i.e., interference signal(s)) to the DAQ (Data Acquisition System) 130. A clock KC is supplied from the OCT light source 101 to the DAQ 130. The clock KC is generated in the OCT light source 101 in synchronization with the output timing of each wavelength sweeping (scanning) within a predetermined wavelength range performed by the wavelength sweeping type light source. For example, the OCT light source 101 optically delays one of the two pieces of branched light obtained by branching the light L0 of each output wavelength, and then generates the clock KC based on the result of the detection of the combined light of the two pieces of branched light. The DAQ 130 performs sampling of the detection result obtained by the detector 125 based on the clock KC. The DAQ 130 sends the sampled detection result(s) obtained by the detector 125 to the image forming unit 70a, the data processor 75a, and the like. The image forming unit 70a (or data processor 75a) applies Fourier transform and the like to the spectral distribution based on the detection result(s) obtained by the detector 125, for example, with respect to a series of wavelength scans (for each A-line) to form the reflection intensity profile in each A-line. In addition, the image forming unit 70a forms image data by applying imaging processing to the reflection intensity profiles of the each A-line.

It should be noted that the difference between the optical path length of the measurement light and the optical path length of the reference light is changed by changing the optical path length of the reference light, in FIG. 9. However, the configuration according to the embodiments is not limited to this. For example, by changing the optical path length of the measurement light, the difference between the optical path length of the measurement light and the optical path length of the reference light may be configured to be changed.

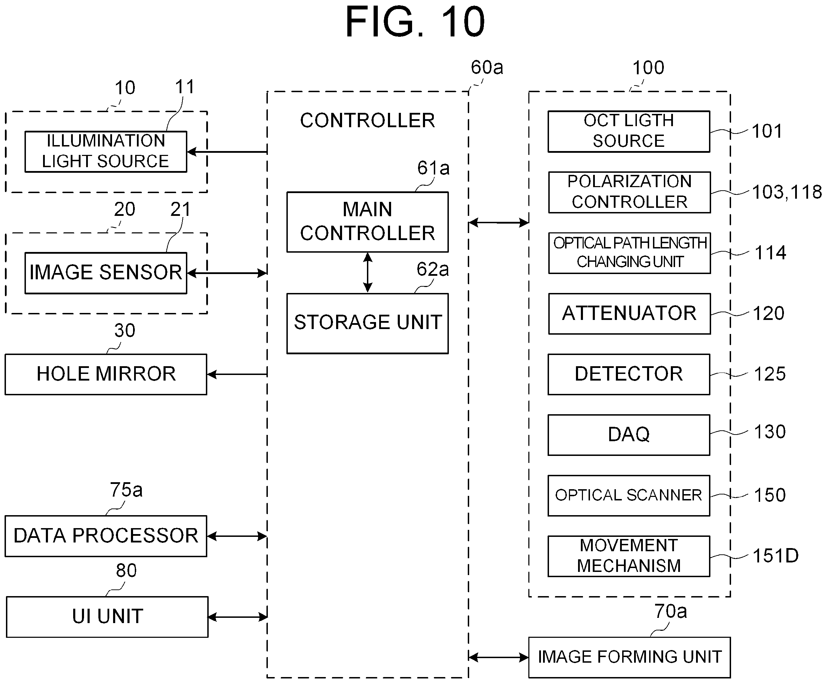

FIG. 10 shows an example of a configuration of a processing system of the fundus observation apparatus 1a according to the second embodiment. In FIG. 10, like reference numerals designate like parts as in FIG. 6, FIG. 8, or FIG. 9. The same description may not be repeated.

The configuration of the processing system of the fundus observation apparatus 1a differs from that of the processing system of the fundus observation apparatus 1 in that a controller 60a is provided instead of the controller 60, that the image forming unit 70a is provided instead of the image forming unit 70, and that the data processor 75a and the OCT optical system 100 are added.

The controller 60a includes a main controller 61a and a storage unit 62a, and in addition to the controls that can be performed by the controller 60, the controller 60a performs control for the image forming unit 70a, the data processor 75a, and the OCT optical system 100. The functions of the main controller 61a are realized by a processor, for example, in the same way as the main controller 61. The storage unit 62a stores, in advance, computer programs for controlling the fundus observation apparatus 1a, in the same way as the storage unit 62. Examples of the computer programs include an illumination light source control program, an image sensor control program, a hole mirror control program, an image forming program, a program for data processing, a program for controlling OCT optical system, a program for user interface. The main controller 61a operates according to the computer programs, and thereby the controller 60a performs the control processing.

The main controller 61a controls each of the slit projection optical system 10, the slit light receiving optical system 20, the hole mirror 30, the image forming unit 70a, the data processor 75a, the OCT optical system 100, and the UI unit 80.

Examples of the control for the OCT optical system 100 include control for the OCT light source 101, operation control for the polarization controllers 103 and 118, movement control for the optical path length changing unit 114, operation control for the attenuator 120, control for the detector 125, control for the DAQ 130, control for the optical scanner 150, and control for a movement mechanism 151D.

Examples of the control for the OCT light source 101 include turning the light source on and off, adjustment of the light amount, adjustment of an aperture, and the like. Examples of the control for the detector 125 include adjustment of exposure of a detecting element, adjustment of gain of a detecting element, adjustment of detecting rate of a detecting element, and the like. Examples of the control for the optical scanner 150 include control of the scan position, the scan range, and scan speed by the optical scanner 150.

The movement mechanism 151D moves the focusing lens 151 in the optical axis direction of the OCT optical system 100. The main controller 61a can control the movement mechanism 151D to move the focusing lens 151 in the optical axis direction of the OCT optical system 100 and to change the focusing position of the measurement light. The focusing position of the measurement light LS corresponds to the depth position (z position) of the beam waist of the measurement light LS.

Examples of the control for the image forming unit 70a include control of forming OCT images based on the detection result(s) of the interference light obtained by the OCT optical system 100, in addition to the image forming control that forms images of the eye E to be examined from the light receiving result(s) obtained using the image sensor 21.

Examples of the data processor 75a include control of image processing for the images formed by the image forming unit 70a and a control of analysis processing for images.

The image forming unit 70a can form the light receiving image (fundus image) corresponding to the opening range (light receivable range) set virtually, based on the light receiving result(s) read out from the image sensor 21, in the same way as the image forming unit 70. The image forming unit 70a can sequentially form light receiving light images corresponding to the virtual opening ranges and can form an image of the eye E to be examined from a plurality of formed light receiving images.

Further, the image forming unit 70a forms image data of the OCT image (tomographic image) based on the detection signals input from the DAQ 130 (detector 125) and pixel position signals input from the controller 60a. Examples of the OCT image formed by the image forming unit 70a include an A-scan image and a B-scan image. The B-scan image is formed by arranging the A-scan images in the B-scan direction. As with the conventional swept source OCT, the image forming processing includes noise removal (noise reduction), filtering, dispersion compensation, fast Fourier transform (FFT), and the like. In the case of employing an OCT apparatus of another type, the image forming unit 70a performs known processing according to the type employed. The various images (the various image data) formed by the image forming unit 70a are stored in the storage unit 62a, for example.

The data processor 75a processes images formed based on the light receiving result(s) obtained by the slit light receiving optical system 20 or data acquired by performing OCT measurement on the eye E to be examined. The data processor 75a can perform various kinds of image processing and various kinds of analysis processing on the image formed by the image forming unit 70a. For example, the data processor 75a performs various types of image correction processing such as brightness correction.

The data processor 75a performs known image processing such as interpolation processing for interpolating pixels between the OCT images to form image data of the three-dimensional image of the fundus Ef. It should be noted that the image data of the three-dimensional image means image data in which the positions of pixels are defined in a three-dimensional coordinate system. Examples of the image data of the three-dimensional image include image data defined by voxels three-dimensionally arranged. Such image data is referred to as volume data or voxel data. In case of displaying an image based on volume data, the data processor 75a performs image rendering processing (e.g., volume rendering, maximum intensity projection (MIP)) on the volume data to form image data of a pseudo three-dimensional image taken from a specific view direction. The pseudo three-dimensional image is displayed on a display device included in the UI unit 80.

Further, the three-dimensional image data may be stack data of a plurality of tomographic images. The stack data is image data formed by three-dimensionally arranging tomographic images along a plurality of scan lines based on positional relationship of the scan lines. That is, the stack data is image data obtained by representing tomographic images, which are originally defined in their respective two-dimensional coordinate systems, by a single three-dimensional coordinate system. That is, the stack data is image data formed by embedding tomographic images into a single three-dimensional space.

The data processor 75a can form a B-mode image (longitudinal cross-sectional image, axial cross-sectional image) in an arbitrary cross section, a C-mode image (transverse section image, horizontal cross-sectional image) in an arbitrary cross section, a projection image, a shadowgram, etc., by performing various renderings on the acquired three-dimensional data set (volume data, stack data, etc.). An image in an arbitrary cross section such as a B-mode image or a C-mode image is formed by selecting pixels (voxels) on a designated cross section from the three-dimensional data set. The projection image is formed by projecting the three-dimensional data set in a predetermined direction (z direction, depth direction, axial direction). The shadowgram is formed by projecting a part of the three-dimensional data set (for example, partial data corresponding to a specific layer) in a predetermined direction. An image having a viewpoint on the front side of the eye to be examined, such as the C-mode image, the projection image, and the shadowgram, is called a front image (en-face image).

The data processor 75a can build (form) the B-mode image or the front image (blood vessel emphasized image, angiogram) in which retinal blood vessels and choroidal blood vessels are emphasized (highlighted), based on data (for example, B-scan image data) acquired in time series by OCT. For example, the OCT data in time series can be acquired by repeatedly scanning substantially the same site of the eye E to be examined.

In some embodiments, the data processor 75a compares the B-scan images in time series acquired by performing B-scan on substantially the same site, converts the pixel value of a change portion of the signal intensity into a pixel value corresponding to the change portion, and builds the emphasized image in which the change portion is emphasized. Further, the data processor 75a forms an OCTA image by extracting information of a predetermined thickness at a desired site from a plurality of built emphasized images and building as an en-face image.

An image (for example, a three-dimensional image, a B-mode image, a C-mode image, a projection image, a shadowgram, and an OCTA image) generated by the data processor 75a is also included in the OCT image.

Further, the data processor 75a performs predetermined analysis processing on the image formed based on the light receiving result(s) obtained by the slit light receiving optical system 20, the detection result of the interference light acquired by the OCT measurement, or the OCT image formed based on the detection result. Examples of the predetermined analysis processing include specifying (identification) of a predetermined site (tissue, lesion) of the eye E to be examined; calculation of a distance, area, angle, ratio, or density between designated sites (distance between layers, interlayer distance); calculation by a designated formula; specifying of the shape of a predetermined site; calculation of these statistics; calculation of distribution of the measured value or the statistics; image processing based on these analysis processing results, and the like. Examples of the predetermined tissue include a blood vessel, an optic disc, a fovea, a macula, and the like. Examples of the predetermined lesion include a leukoma, a hemorrhage, and the like.

The fundus observation apparatus 1a may include a movement mechanism that moves the OCT optical system 100 in a one-dimensional direction or predetermined two-dimensional directions intersecting the optical axis of the OCT optical system 100. In this case, the main controller 61a controls this movement mechanism to move the OCT optical system 100 relative to the dichroic mirror 90 in a one-dimensional direction or two-dimensional directions intersecting the optical axis of the OCT optical system 100. This allows to move the scan range using the optical scanner 150 in the OCT optical system 100 and to scan the wide-angle scan range (e.g., imaging range of SLO) on the fundus Ef.

The OCT optical system 100 is an example of a "projection optical system" and a "light receiving optical system" according to the embodiments.

### <Operation>

Next, an example of the operation of the fundus observation apparatus 1a according to the second embodiment will be described.

The fundus observation apparatus 1a can perform OCT measurement using the OCT optical system 100 in parallel with the scan control for the fundus Ef using the illumination light shown in FIG. 7. Hereafter, the control of OCT measurement that can be performed in parallel with the control shown in FIG. 7 will be described.

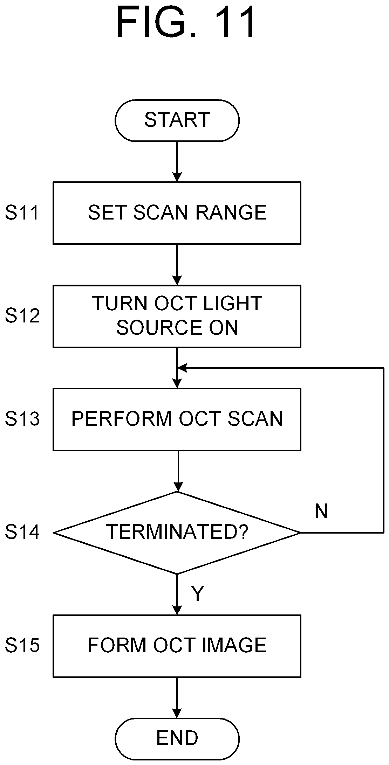

FIG. 11 shows an example of an operation of the fundus observation apparatus 1a according to the second embodiment. FIG. 11 represents a flowchart of the example of the operation of the fundus observation apparatus 1a according to the second embodiment. The storage unit 62a stores computer program(s) for realizing the processing shown in FIG. 11. The main controller 61a operates according to the computer programs, and thereby the main controller 61a performs the processing shown in FIG. 11.

It is assumed that the eye E to be examined is arranged at a predetermined eye to be examined position (the second focal point F4 of the second ellipsoidal mirror 50 in FIG. 1), in FIG. 11.

### (S1: Set scan range)

First, the main controller 61a sets the scan range of the optical scanner 150. The main controller 61a can set the scan start position, the scan end position, the scan speed (scan frequency), etc. by the optical scanner 150, along with the scan range.

In some embodiments, the user can designate the scan mode or the operation mode by operating the operation device in the UI unit 80. When the scan mode (for example, horizontal scan, vertical scan) is designated by operating the operation device by the user, the main controller 61a analyzes operation information from the operation device to identify the designated scan mode. When the operation mode is designated by operating the operation device by the user, the main controller 61a analyzes the operation information to identify a scan mode (for example, horizontal scan, vertical scan) designated in advance in the designated operation mode (for example, OCT measurement mode).

### (S12: Turn OCT light source on)

Subsequently, the main controller 61a controls the OCT light source 101 to turn the OCT light source 101 on. In some embodiments, the main controller 61a performs step S12 in synchronization with the turning on control of the illumination light source 11 in step S1 shown in FIG. 7.

In some embodiments, the main controller 61a performs controls of adjusting focusing and of adjusting polarization. For example, the main controller 61a controls the OCT optical system 100 to perform OCT measurement, after controlling the movement mechanism 151D to move the focusing lens by a predetermined distance. The main controller 61a controls the data processor 75a to determine the focus state of the measurement light LS based on the detection result of the interference light acquired by the OCT measurement. For example, the data processor 75a analyzes the detection results of the interference light acquired by the OCT measurements to calculate predetermined evaluation value(s) relating to the image quality of OCT images and to determine the focus state based on the calculated evaluation value(s). When it is determined that the focus state is not appropriate based on the determination result of the data processor 75a, the main controller 61a controls a movement mechanism 151D again and repeats this until it is determined that the focus state of the measurement light LS is appropriate.

Further, for example, the main controller 61a controls the OCT optical system 100 to perform OCT measurement after controlling at least one of the polarization controller 103 or the polarization controller 118 to change the polarization state of at least one of the light L0 or the measurement light LS by a predetermined amount. And then, the main controller 61a controls the image forming unit 70a to form the OCT image on the basis of the acquired detection result of the interference light. The main controller 61a controls the data processor 75a to determine the image quality of the OCT image acquired by performing the OCT measurement. When it is determined that the polarization state of the measurement light LS is not appropriate based on the determination result of the data processor 75a, the main controller 61a controls the polarization controllers 103 and 118 again and repeats this until it is determined that the polarization state of the measurement light LS is appropriate.

### (S13: Perform OCT scan)

Subsequently, the main controller 61a controls the optical scanner 150 to deflect the measurement light LS generated based on the light L0 emitted from the OCT light source 101 to scan a predetermined site on the fundus Ef of the eye E to be examined with the deflected measurement light LS. The detection result of the interference light acquired by the OCT measurement is sampled by the DAQ 130 and is stored as the interference signal in the storage unit 62a or the like.

### (S14: Terminated?)

Subsequently, the main controller 61a determines whether or not to terminate the OCT scan for the fundus Ef. For example, the main controller 61a can determine whether or not to terminate the OCT scan for the fundus Ef, by determining whether or not the deflection angle of the deflection surface of the optical scanner 150, which is changed sequentially, is within the predetermined deflection angle range.

When it is determined to terminate the OCT scan for the fundus Ef (S14: Y), the operation of the fundus observation apparatus 1a proceeds to step S15. When it is determined not to terminate the OCT scan for the fundus Ef (S14: N), the operation of the fundus observation apparatus 1a proceeds to step S13.

### (S15: Form OCT image)

When it is determined in step S14 to terminate the OCT scan for the fundus Ef (S14: Y), the main controller 61a controls the image forming unit 70a to form a plurality of A-scan images of the fundus Ef along the B-scan direction, based on the interference signal acquired in step S14. In some embodiments, the main controller 61a controls the data processor 75a to form OCT images such as three-dimensional OCT images, B-mode images, C-mode images, projection images, shadowgrams, and OCTA images.

This terminates the operation of the fundus observation apparatus 1a.

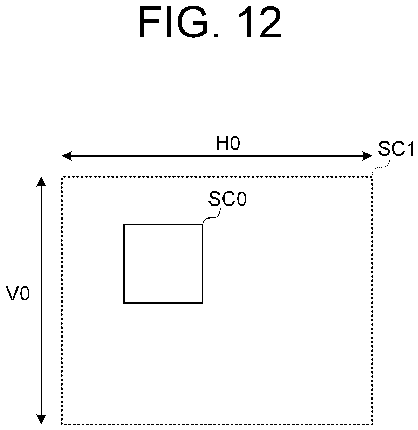

FIG. 12 shows a diagram explaining the operation of the fundus observation apparatus 1a according to the second embodiment.

As shown in FIG. 12, the scan using the illumination light on the fundus Ef, which is realized by deflecting the illumination light using the hole mirror 30, and the OCT scan on the fundus Ef, which is realized by deflecting the measurement light LS using the optical scanner 150, are performed in parallel. Thereby, when the scan using the illumination light is being performed in a scan range SC1 (horizontal direction H0 × vertical direction V0) on the fundus Ef, the OCT scan can be performed on the scan range SC0 at any position within the scan range SC1.

As a result, the OCT measurement (OCT imaging) can be performed on any position of the fundus being observed at a large wide angle by scanning using the illumination light for the scan range SC1.

As described above, according to the second embodiment, in addition to the effects obtained by the first embodiment, by optically coupling the OCT optical system 100 on the transmission side of the hole mirror 30 as the deflecting member (through the hole in the hole mirror), the optical path of the wide-angle illumination light and the optical path of the returning light of the illumination light can be separated at low cost. Further, OCT measurement (OCT imaging) can be performed on any position of the fundus being observed at a large wide angle, without sharing an optical scanner for OCT scanning and an optical scanner for deflection of illumination light.

### <Third embodiment>

In the first embodiment and the second embodiment, the case where the second ellipsoidal mirror 50 is arranged so that the angle α between the straight line connecting the first focal point F1 and the second focal point F2 of the first ellipsoidal mirror 40 and the straight line connecting the first focal point F3 and the second focal point F4 of the second ellipsoidal mirror 50 is 30 degrees has been described. However, the configuration according to the embodiments is not limited to this. For example, the angle α between the straight line connecting the first focal point F1 and the second focal point F2 of the first ellipsoidal mirror 40 and the straight line connecting the first focal point F3 and the second focal point F4 of the second ellipsoidal mirror 50 may be an approximate 0 degree.

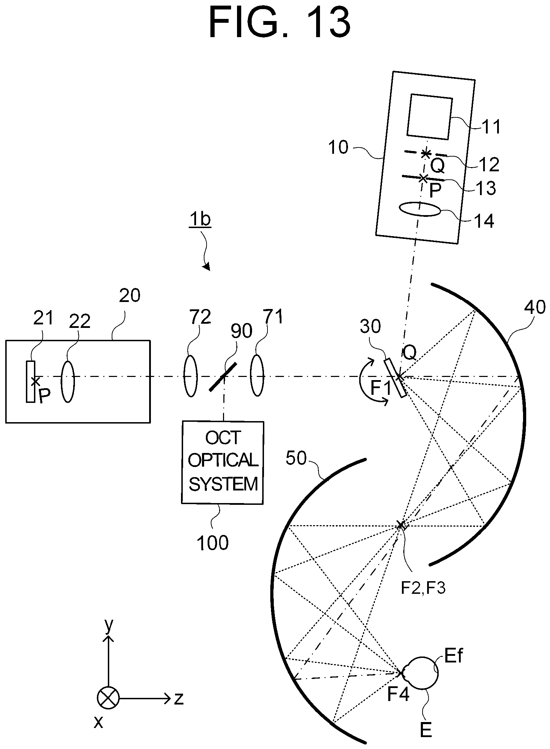

FIG. 13 shows an example of a configuration of an optical system of the fundus observation apparatus according to the third embodiment. In FIG. 13, like parts are designated by like reference numerals as in FIG. 8 and repetitious description of such parts may not be provided.

The difference between the configuration of the optical system of the fundus observation apparatus 1b according to the third embodiment and the that of the optical system of the fundus observation apparatus 1a according to the second embodiment is the arrangement of the second ellipsoidal mirror 50 relative to the first ellipsoidal mirror 40. In the fundus observation apparatus 1b, the second ellipsoidal mirror 50 is arranged so that the angle α between the straight line connecting the first focal point F1 and the second focal point F2 of the first ellipsoidal mirror 40 and the straight line connecting the first focal point F3 and the second focal point F4 of the second ellipsoidal mirror 50 is 0.2 degrees (approximate 0 degree).

It should be noted that the fundus observation apparatus 1b is provided with the OCT optical system 100, in FIG. 13. However, the fundus observation apparatus 1b may have a configuration in which the OCT optical system 100 is omitted, in the same way as in FIG. 1.

Depending on the angle α described above, the symmetry of the observation range for the eye E to be examined changes along with a range of wide angle. According to the third embodiment, the fundus Ef can be observed in a wide-angle range that is symmetrical to the eye E to be examined, compared to the second embodiment.

### <Fourth embodiment>

In the first embodiment, the case where the illumination light is deflected using the hole mirror 30 has been described. However, the configuration of the embodiments is not limited thereto. In the first embodiment, for example, a reflective mirror may be placed at the first focal point F1 of the first ellipsoidal mirror 40 and the hole mirror may be placed at a position substantially conjugate optically to the pupil of the eye E to be examined.

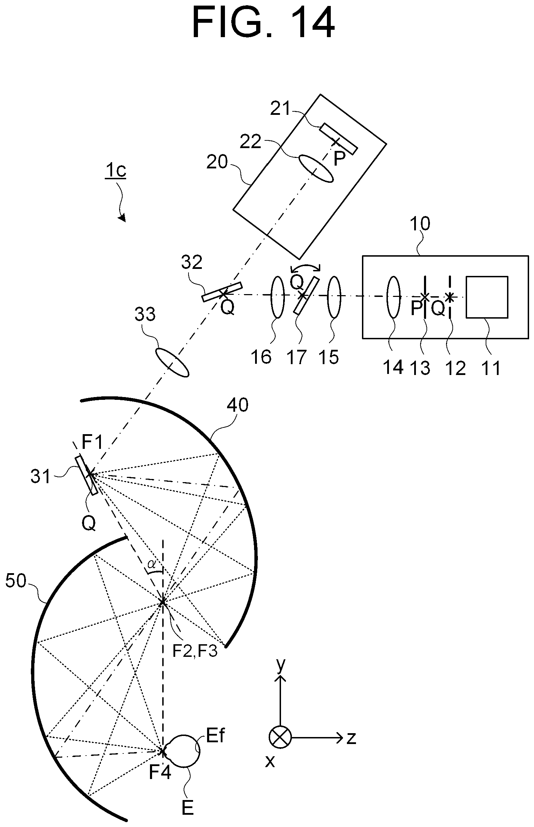

FIG. 14 shows an example of a configuration of an optical system of the fundus observation apparatus according to the fourth embodiment. In FIG. 14, like parts are designated by like reference numerals as in FIG. 1 and repetitious description of such parts may not be provided.

The configuration of the optical system of the fundus observation apparatus 1c according to the fourth embodiment differs from that of the optical system of the fundus observation apparatus 1 according to the first embodiment in that a reflective mirror 31 is disposed at the first focal point F1 of the first ellipsoidal mirror 40 instead of the hole mirror 30, that the hole mirror 32 is disposed at the pupil conjugate position Q away from the first focal point F1, that an optical scanner 17 is disposed between the hole mirror 32 and the slit projection optical system 10, and that relay lenses 33, 15, and 16 for relaying the pupil conjugate position Q are added.

The orientation of the deflection surface of the reflective mirror 31 is fixed. The relay lens 33 is arranged between the reflective mirror 31 and the hole mirror 32. The hole mirror 32 separates or couples the optical path of the slit projection optical system 10 and the optical path of the slit light receiving optical system 20. The orientation of the deflection surface of the hole mirror 32 is fixed. The relay lens 16, the optical scanner 17, and the relay lens 15 are arranged between the hole mirror 32 and the slit projection optical system 10. The optical scanner 17 is a uniaxial scanner that performs deflection operation of the illumination light in the same way as the hole mirror 30.

In this case, the illumination light from the slit projection optical system 10 passes through the relay lens 15, and is deflected by the optical scanner 17. The illumination light deflected by the optical scanner 17 passes through the relay lens 16, and is deflected on a peripheral region of the hole formed in the hole mirror 32 to be guided to the relay lens 33. The illumination light that has been guided to the relay lens 33 is reflected by the reflective mirror 31 and is guided to the reflective surface of the first ellipsoidal mirror 40. The returning light of the illumination light from the eye E to be examined is deflected by the reflective mirror 31, passes through the relay lens 33, passes through the hole of the hole mirror 32, and is guided to the slit light receiving optical system 20.

According to the fourth embodiment, compared to the first embodiment, the degree of freedom in the arrangement of the slit projection optical system 10 and the slit light receiving optical system 20 can be improved by relaying the pupil conjugate position Q, even if there is no room for an optical system in the vicinity of the first focal point F1 of the first ellipsoidal mirror 40.

### <Fifth embodiment>

In the second embodiments, the case where the illumination light is deflected using the hole mirror 30 has been described. However, the configuration according to the embodiments is not limited thereto. In the second embodiment, as in the fourth embodiment, for example, the reflective mirror may be placed at the first focal point F1 of the first ellipsoidal mirror 40, and the hole mirror may be placed at a position substantially conjugate optically to the pupil of the eye E to be examined.

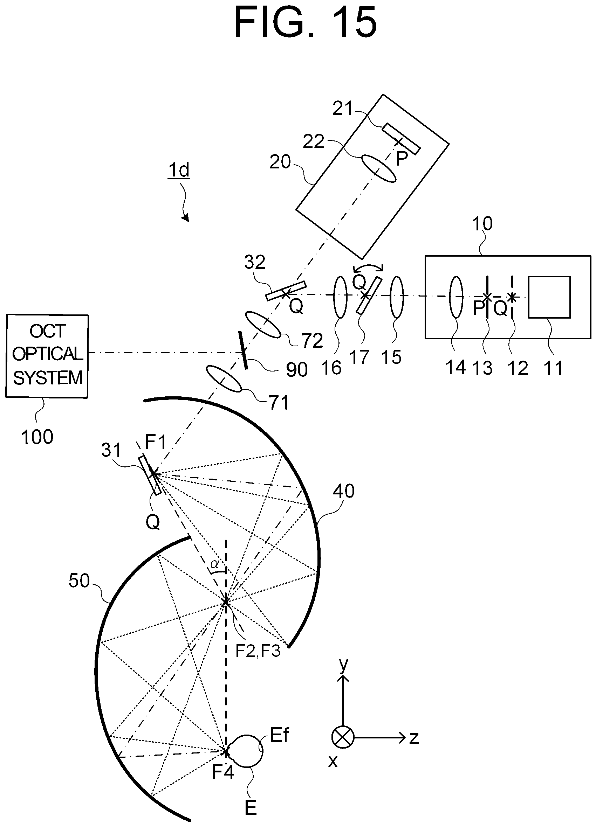

FIG. 15 shows an example of a configuration of an optical system of the fundus observation apparatus according to the fifth embodiment. In FIG. 15, like reference numerals designate like parts as in FIG. 8 or FIG. 14. The same description may not be repeated.

The configuration of the optical system of the fundus observation apparatus 1d according to the fifth embodiment differs that of the optical system of the fundus observation apparatus 1a according to the second embodiment in that the reflective mirror 31 is disposed at the first focal point F1 of the first ellipsoidal mirror 40 instead of the hole mirror 30, that the hole mirror 32 is disposed at the pupil conjugate position Q away from the first focal point F1, that the optical scanner 17 is disposed between the hole mirror 32 and the slit projection optical system 10, and that relay lenses 15 and 16 for relaying the pupil conjugate position Q are added.

The orientation of the deflection surface of the reflective mirror 31 and the orientation of the deflection surface of the hole mirror 32 are fixed, in the same way as in the fourth embodiment. The pupil conjugate position Q is relayed by relay lenses 71 and 72. The hole mirror 32 separates or couples the optical path of the slit projection optical system 10 and the optical path of the slit light receiving optical system 20. The relay lens 16, the optical scanner 17, and the relay lens 15 are arranged between the hole mirror 32 and the slit projection optical system 10. The optical scanner 17 is a uniaxial scanner that performs deflection operation of the illumination light in the same way as the hole mirror 30.

In this case, the illumination light from the slit projection optical system 10 passes through the relay lens 15, and is deflected by the optical scanner 17. The illumination light deflected by the optical scanner 17 passes through the relay lens 16, is deflected on the peripheral region of the hole formed in the hole mirror 32, passes through the relay lens 72, the dichroic mirror 90, and the relay lens 71, is reflected by the reflective mirror 31, and is guided to the reflective surface of the first ellipsoidal mirror 40. The returning light of the illumination light from the fundus Ef of the eye E to be examined is deflected by the reflective mirror 31, passes through the relay lens 71, the dichroic mirror 90, and the relay lens 72, passes through the hole of the hole mirror 32, and is guided to the slit light receiving optical system 20.

According to the fifth embodiment, compared to the second embodiment, the degree of freedom in the arrangement of the slit projection optical system 10 and the slit light receiving optical system 20 can be improved by relaying the pupil conjugate position Q, even if there is no room for an optical system in the vicinity of the first focal point F1 of the first ellipsoidal mirror 40.

### [Actions]

The fundus observation apparatus according to the embodiments will be described.

The first aspect of the embodiments is a fundus observation apparatus (1, 1a, 1b, 1c, 1d). The fundus observation apparatus includes: an optical system (slit projection optical system 10 and light receiving optical system 20, OCT optical system 100) configured to project light from a light source (illumination light source 11, OCT light source 101) onto a fundus (Ef) of an eye (E) to be examined, and to receive returning light of the illumination light from the fundus; two concave mirrors (first ellipsoidal mirror 40, second ellipsoidal mirror) having concave reflective surfaces, respectively, that guide the light from the optical system to the fundus and guide the returning light to the optical system; and a holding member (45) configured to be capable of holding the two concave mirrors. The holding member 45 is configured to be capable of holding the two concave mirrors in a state where the concave mirrors are arranged on both sides of the holding member so that the concave mirrors are positioned in a default relative position in predetermined one-dimensional direction or predetermined two-dimensional directions.

According to such an aspect, since the two concave mirrors are fixed in a predetermined positional relationship for one-dimensional direction or two-dimensional directions among three-dimensional directions, the number of directions in which the positional relationship of the two concave mirrors should be adjusted can be reduced. Thereby, the accuracy of fundus observation can be improved at a low cost while easily achieving optical measurement conditions designed for high precision. In particular, by manufacturing optical members such as concave mirrors using high-precision machining techniques, the deterioration of optical measurement accuracy caused by positioning accuracy can be suppressed.

In the second aspect of the embodiments, in the first aspect, the holding member is configured to hold the two concave mirrors on both sides at a default distance in an optical axis direction of the optical system.

According to such an aspect, by merely adjusting the two concave mirrors in the two-dimensional directions that intersects the optical axis direction of the optical system, the optical measurement conditions designed for high precision can be easily achieved.

In the third aspect of the embodiments, in the first aspect or the second aspect, the two concave mirrors further includes: a first concave mirror (first ellipsoidal mirror 40) having a first flange on a periphery and having a concave first reflected surface that reflects the light; and a second concave mirror (second ellipsoidal mirror 50) having a second flange on a periphery and having a concave second reflected surface that guides light reflected on the first concave mirror to the fundus. The holding member is configured to hold the first concave mirror and the second concave mirror so that the first flange and the second flange are approximately parallel to each other.

According to such an aspect, by moving at least one of the first concave mirror or the second concave mirror parallel to each other, the two concave mirrors can be easily adjusted, such as position matching of the two concave mirrors, with high precision.

In the fourth aspect of the embodiments, in any one of the fist aspect to the third aspect, both ends in a predetermined first direction (direction of the major axis of the ellipsoidal mirror) of at least one of the two concave mirrors have a shape cut in a plane intersecting the first direction.

According to such an aspect, the weight and the size of the fundus observation apparatus can be reduced while securing the size of the reflective surface necessary for wide-angle fundus observation. In particular, this allows the examinee's mouth or chin to avoid interfering with the concave mirror(s). Thereby, the eye to be examined can be observed with the examinee facing forward to the reflective surface.

In the fifth aspect of the embodiments, in any one of the first aspect to the fourth aspect, at least one of the two concave mirrors is an ellipsoidal mirror (first ellipsoidal mirror 40, second ellipsoidal mirror 50).

According to such an aspect, the fundus observation apparatus can be provided in which the position matching of the ellipsoid mirrors can be easily performed with high precision.

In the sixth aspect of the embodiments, in any one of the first aspect to the fourth aspect, the two concave mirrors further include a first ellipsoidal mirror (40) and a second ellipsoidal mirror (50). One (second focal point F2) of two focal points of the first ellipsoidal mirror is located at one (first focal point F3) of two focal points of the second ellipsoidal mirror, and the light from the optical system is configured to be guided to the other (second focal point F4) of the two focal points of the second ellipsoidal mirror.

According to such an aspect, in the fundus observation apparatus using the two ellipsoidal mirrors, the concave mirrors can be performed position matching at low cost and with high precision.

In the seventh aspect of the embodiments, in the sixth aspect, the optical system further includes: a projection optical system (slit projection optical system 10, OCT optical system 100) configured to project light from the light source; a light receiving optical system (slit light receiving optical system 20, OCT optical system 100) configured to receive the returning light; and a deflecting member (hole mirror 30) located at the other (first focal point F1) of the two focal points of the first ellipsoidal mirror and configured to deflect the light from the light source and to guide the returning light to the light receiving optical system.

According to such an aspect, a shared optical system shared between the optical path of the wide-angle illumination light and the optical path of the returning light can be easily arranged while ensuring an angle of view for imaging of more than 80 degrees with the optical system alone, with a low cost and compact configuration.

In the eighth aspect of the embodiments, in the sixth aspect, the optical system further includes: a projection optical system (slit projection optical system 10) including a deflecting member (optical scanner 17) and configured to deflect light from the light source to project; a light receiving optical system (slit light receiving optical system 20) configured to receive the returning light; and an optical path coupling member (hole mirror 32) configured to optically couple an optical path of the projection optical system with an optical path of the light receiving optical system; and a reflective member (reflective mirror 31) located at the other (first focal point F 1) of the two focal points of the first ellipsoidal mirror and configured to guide the light from the light source to the first ellipsoidal mirror, the light having been guided through an optical path coupled by the optical path coupling member.

According to such an aspect, the degree of freedom in the arrangement of the slit projection optical system and the slit light receiving optical system can be improved, even if there is no room for an optical system in the vicinity of the second focal point of the first ellipsoidal mirror.

### < Others >

The above-described embodiments are merely examples for carrying out the present invention. Those who intend to implement the present invention can apply any modification, omission, addition, or the like within the scope of the gist of the present invention.

In some embodiments, a program for causing a processor (computer) to execute the method of controlling the fundus observation apparatus described above is provided. Such a program can be stored in any non-transitory recording medium (storage medium) that can be read by a computer. Examples of the recording medium include a semiconductor memory, an optical disk, a magneto-optical disk (CD-ROM, DVD-RAM, DVD-ROM, MO, etc.), a magnetic storage medium (hard disk, floppy (registered trade mark) disk, ZIP, etc.), and the like. The computer program may be transmitted and received through a network such as the Internet, LAN, etc.

### [EXPLANATION OF SYMBOLS]

1, 1a, 1b, 1c, 1d Fundus observation apparatus
10 Slit projection optical system
17, 150 Optical scanner
20 Slit light receiving optical system
30, 32 Hole mirror
31 Reflective mirror
40 First ellipsoidal mirror
45 Holding member
50 Second ellipsoidal mirror
100 OCT optical system
E Eye to be examined
F1, F3 First focal point
F2, F4 Second focal point
P Fundus conjugate position
Q Pupil conjugate position

## Claims

1. A fundus observation apparatus, comprising:
an optical system configured to project light from a light source onto a fundus of an eye to be examined, and to receive returning light from the fundus;
two concave mirrors having concave reflective surfaces, respectively, configured to guide the light from the optical system to the fundus, and to guide the returning light to the optical system; and
a holding member configured to be capable of holding the two concave mirrors in a state where the concave mirrors are arranged on both sides of the holding member so that the concave mirrors are positioned in a default relative position in a predetermined one-dimensional direction or predetermined two-dimensional directions.

2. The fundus observation apparatus of claim 1, wherein
the holding member is configured to hold the two concave mirrors on both sides of the holding member at a default distance in an optical axis direction of the optical system.

3. The fundus observation apparatus of claim 1 or 2, wherein
the two concave mirrors further include:
a first concave mirror having a first flange on a periphery and having a concave first reflected surface that reflects the light; and
a second concave mirror having a second flange on a periphery and having a concave second reflected surface that guides light reflected on the first concave mirror to the fundus, and
the holding member is configured to hold the first concave mirror and the second concave mirror so that the first flange and the second flange are approximately parallel to each other.

4. The fundus observation apparatus of any one of claims 1 to 3, wherein
both ends in a predetermined first direction of at least one of the two concave mirrors have a shape cut in a plane intersecting the first direction.

5. The fundus observation apparatus of any one of claims 1 to 4, wherein
at least one of the two concave mirrors is an ellipsoidal mirror.

6. The fundus observation apparatus of any one of claims 1 to 4, wherein
the two concave mirrors further include a first ellipsoidal mirror and a second ellipsoidal mirror,
one of two focal points of the first ellipsoidal mirror is located at one of two focal points of the second ellipsoidal mirror, and
the two concave mirrors are configured to guide the light from the optical system to the other of the two focal points of the second ellipsoidal mirror.

7. The fundus observation apparatus of claim 6, wherein
the optical system further includes:
a projection optical system configured to project light from the light source;
a light receiving optical system configured to receive the returning light; and
a deflecting member located at the other of the two focal points of the first ellipsoidal mirror and configured to deflect the light from the light source and to guide the returning light to the light receiving optical system.

8. The fundus observation apparatus of claim 6, wherein
the optical system further includes:
a projection optical system including a deflecting member and configured to deflect light from the light source to project;
a light receiving optical system configured to receive the returning light; and
an optical path coupling member configured to optically couple an optical path of the projection optical system with an optical path of the light receiving optical system; and
a reflective member located at the other of the two focal points of the first ellipsoidal mirror and configured to guide the light from the light source to the first ellipsoidal mirror, the light having been guided through an optical path coupled by the optical path coupling member.
